# EUROPEAN PATENT APPLICATION

(11) **EP 4 261 209 A1**
(43) Date of publication of application: **18.10.2023**
(21) Application number: 21903252.1
(22) Date of filing: 30.11.2021
(51) Int. Cl.: C07D 277/64, C12N 15/11, C12Q 1/68, G01N 21/64, G01N 21/78

(54) **NUCLEIC ACID DETECTION METHOD, COMPOUND AND FLUORESCENT PROBE**

(30) Priority: 11.12.2020 JP 2020206368
(71) Applicant: Wakayama University, Wakayama-shi, Wakayama 640-8510 (JP)
(72) Inventor: SAKAMOTO Takashi, Wakayama-shi, Wakayama 640-8510 (JP)
(74) Representative: RGTH
(86) International application number: PCT/JP2021/043996
(87) International publication number: WO 2022/124148

(57) **Abstract**

A compound suitable for nucleic acid detection or viscosity measurement is provided. A compound of the disclosure includes a donor and three acceptors bound to the donor so as to perform rotary motion with respect to the donor. A fluorescence property of the compound changes in accordance with change in the rotary motion of each acceptor.

## Description

### TECHNICAL FIELD

The present disclosure relates to a nucleic acid detection method, a compound, and a fluorescent probe. This application claims priority on Japanese Patent Application No. 2020-206368 filed on December 11, 2020, the entire content of which is incorporated herein by reference.

### BACKGROUND ART

PATENT LITERATURE 1 discloses a compound as a DNA probe. A representative compound disclosed in PATENT LITERATURE 1 is quinone cyanine-dithiazole (QCy-DT).

QCy-DT is a "donor-two-acceptor (D2A)" π-electronic system in which two acceptors are bound to one donor. QCy-DT is one class belonging to D2A fluorophore (Qcy7).

QCy-DT includes a donor phenol moiety and two heterocyclic electron acceptors bound to the donor phenol moiety.

QCy-DT has a molecular structure in a curved shape and can bind to a minor groove of a double-stranded DNA. Upon binding to a double-stranded DNA, QCy-DT is converted into a switch-on near-infrared fluorophore through internal charge transfer. That is, upon binding to a double-stranded DNA, QCy-DT emits fluorescence. QCy-DT is used as a fluorescent probe for detection of double-stranded DNAs.

Similar to PATENT LITERATURE 1, NON PATENT LITERATURE 1 discloses QCy-DT. NON PATENT LITERATURE 2 discloses a "donor-two-acceptor (D2A)" dye. NON PATENT LITERATURE 2 discloses various examples of potential acceptor moieties.

### CITATION LIST

### [PATENT LITERATURE]

PATENT LITERATURE 1: US Patent No. 10,683,273

### [NON PATENT LITERATURE]

NON PATENT LITERATURE 1: Nagarjun Narayanaswamy et al, Sequence-specific recognition of DNA minor groove by an NIR-fluorescence switch-on probe and its potential applications, Nucleic Acids Research, 2015 Vol. 43, No. 18, pp. 8651-8663
NON PATENT LITERATURE 2: Naama Karton-Lifshin et al, Donor-Two-Acceptor" Dye Design: A Distinct Gateway to NIR Fluorescence, Journal of the American Chemical Society, 2012, pp.20412-20420

### SUMMARY OF INVENTION

QCy-DT cannot detect a quadruplex nucleic acid. In addition, none of the afore-mentioned literatures discloses detection of a quadruplex nucleic acid nor detection of a double-stranded nucleic acid and a quadruplex nucleic acid so as to be distinguished from each other. Further, none of the literatures discloses measurement of viscosity by a fluorescent probe. It is desired to solve at least one of these problems.

An aspect of the present disclosure is a method including using a compound for nucleic acid detection. The compound used in the method of the disclosure has a structure represented by general formula (1A) or general formula (1B) according to an embodiment described later.

Another aspect of the present disclosure is a compound. The compound of the disclosure has a structure represented by general formula (1A) or general formula (1B) according to the embodiment described later.

Another aspect of the present disclosure is a fluorescent probe. The fluorescent probe of the disclosure includes the compound described above.

Another aspect of the present disclosure is a method including using the compound described above for measurement of a viscosity of a liquid.

The compound of the present disclosure may be a compound including: a donor; and three acceptors bound to the donor so as to perform rotary motion with respect to the donor, wherein fluorescence from the compound changes in accordance with change in the rotary motion of each acceptor. A method of the present disclosure may be a method including using a compound including: a donor; and three acceptors bound to the donor so as to perform rotary motion with respect to the donor, wherein fluorescence from the compound changes in accordance with change in the rotary motion of each acceptor. The using of the compound may be for nucleic acid detection or for measurement of a viscosity of a liquid.

Further details will be described later as an embodiment.

### BRIEF DESCRIPTION OF DRAWINGS

FIG. 1 illustrates a donor-three-acceptor structure and nucleic acid detection.
FIG. 2 illustrates a donor-two-acceptor structure and nucleic acid detection.
FIG. 3 illustrates compounds according to an embodiment.
FIG. 4 illustrates a production method of a compound according to the embodiment.
FIG. 5 shows glycerol-mixing-ratio dependency of fluorescence spectra (excitation wavelength: 540 nm) of the compound (6 µM) according to the embodiment.
FIG. 6 shows correlation between fluorescence intensity (680 nm) of the compound (6 µM) according to the embodiment and the mixing ratio of glycerol.
FIG. 7 shows correlation between fluorescence intensity (680 nm) of the compound (6 µM) according to the embodiment and solvent viscosity.
FIG. 8 shows glycerol-mixing-ratio dependency of excitation spectra (detection wavelength: 680) of the compound (6 µM) according to the embodiment.
FIG. 9 is a diagram obtained by normalizing the fluorescence intensity in FIG. 8.
FIG. 10 shows correlation between fluorescence intensity ratio (540 nm/470 nm) of the compound according to the embodiment and the mixing ratio of glycerol.
FIG. 11 shows correlation between fluorescence intensity ratio (540 nm/470 nm) of the compound according to the embodiment and solvent viscosity.
FIG. 12 shows glycerol-mixing-ratio dependency of absorption spectra of the compound (6 µM) according to the embodiment.
FIG. 13 shows correlation between the absorption local maximum of the compound according to the embodiment and the mixing ratio of glycerol.

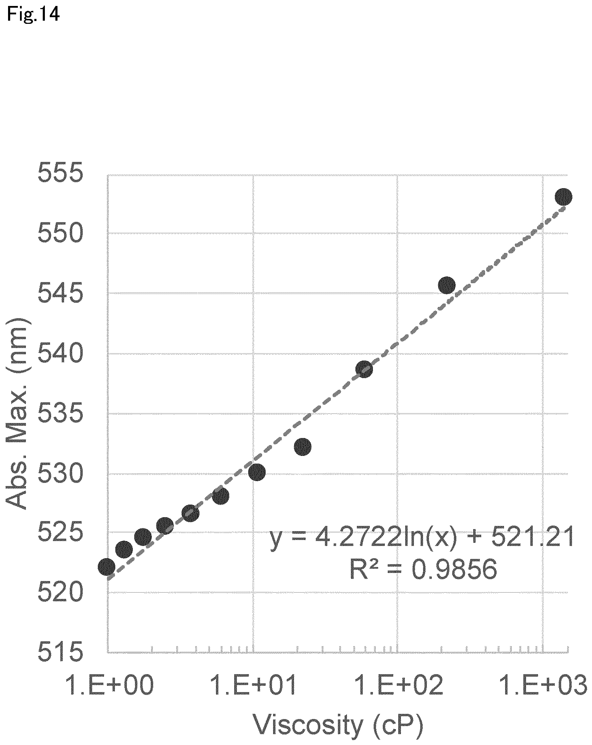

FIG. 14 shows correlation between the absorption local maximum of the compound according to the embodiment and solvent viscosity.
FIG. 15 shows fluorescence spectra when various oligo DNAs were added (excitation wavelength: 470 nm, [the compound according to the embodiment]=[DNA]=6 µM in 50 mM Tris-HCL (pH 7.5), 100 mM KCL).
FIG. 16 shows fluorescence spectra when various oligo DNAs were added (excitation wavelength: 570 nm, [the compound according to the embodiment]=[DNA]=6 µM in 50 mM Tris-HCL (pH 7.5), 100 mM KCL).
FIG. 17 shows 700 nm fluorescence intensity change when a quadruplex DNA (Tel26, Telo_G4, MycG4) was added to the compound according to the embodiment.
FIG. 18 shows 700 nm fluorescence intensity change when a double-stranded DNA (ds(A/T)₄(T/A)₄, ds(AAATTT)) was added to the compound according to the embodiment.
FIG. 19 is images showing results of staining of fixed cells by the compound according to the embodiment.
FIG. 20 shows fluorescence intensities and intensity differences in cells shown in FIG. 19.
FIG. 21 shows relationship between addition concentration of the compound according to the embodiment and viable cell percentage.
FIG. 22 shows results of staining of viable cells (HeLa cells) by the compound according to the embodiment.
FIG. 23 shows fluorescence intensities and intensity differences in cells shown in FIG. 22.
FIG. 24 is images of results of fluorescence microscopy, showing response to a quadruplex stabilization reagent (Pyridostatin (PDS)) of viable cells stained by the compound according to the embodiment.
FIG. 25 is dot plots obtained by quantifying response (fluorescence intensity change) to PDS of viable cells (HeLa, A549, HEK293, MRC-5).
FIG. 26 is photographs showing presence or absence of precipitation according to the types of solvents for a staining liquid (probe solution).
FIG. 27 is images showing results of fluorescence microscopy (fixed cells), showing results of competition experiments with Hoechist 33258.
FIG. 28 is images showing results of fluorescence microscopy (viable cells), showing results of competition experiments with Hoechist 33258.

### DESCRIPTION OF EMBODIMENTS

### <1. Overview of nucleic acid detection method, compound, and fluorescent probe>

(1) A method according to an embodiment includes using a compound having a structure represented by general formula (1A) or general formula (1B), for nucleic acid detection. When this compound binds to a double-stranded nucleic acid or a quadruplex nucleic acid, the fluorescence property of the compound changes. Therefore, this compound is useful for nucleic acid detection, for example. 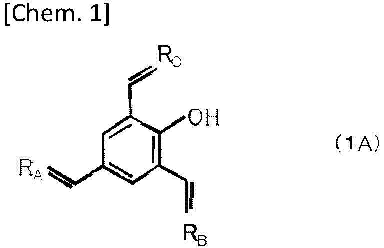 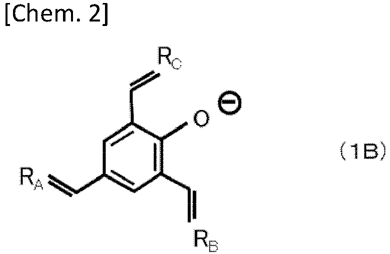 Here,
   R_{A}, R_{B}, and R_{C} each independently have a structure selected from the group consisting of formula (1C), formula (1D), formula (1E), and formula (1F) below (* representing a binding site), 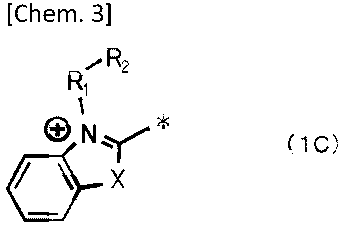 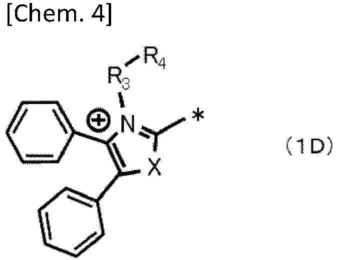 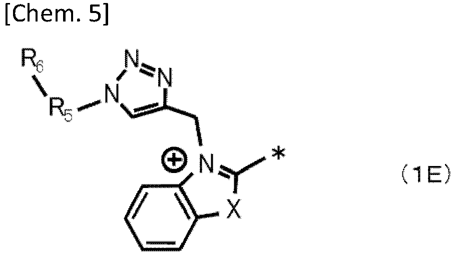 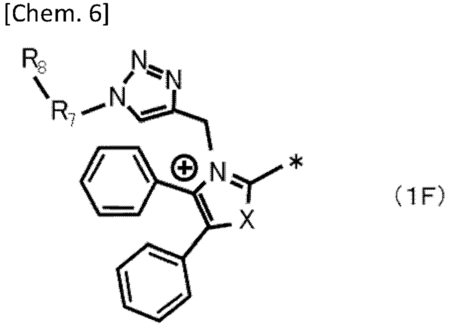
   X is selected from the group consisting of O, S, and Se,
   R₁, R₃, R₅, and R₇ are each independently selected from the group consisting of H, -(CH₂)n-, and -(CH₂CH₂O)n-, n being a natural number of 1 to 100, and
   R₂, R₄, R₆, and R₈ are each independently selected from the group consisting of H, -OH, methyl, an amine, a terminal alkyne, an alkene, an alkyl acid, an amine acid, sulfonate (SO₃⁻), an aryl group or a derivative thereof, and a heterocyclic compound or a derivative thereof.
(2) Preferably, the method includes using the compound for detection of at least either one of a double-stranded nucleic acid and a quadruplex nucleic acid.
(3) Preferably, the method includes using the compound for nucleic acid detection in which a double-stranded nucleic acid and a quadruplex nucleic acid are distinguished from each other.
(4) Preferably, the method includes using the compound for detection of a viscosity of a sample containing a nucleic acid.
(5) Preferably, X is S.
(6) A compound according to the embodiment has a structure represented by general formula (1A) or general formula (1B). When this compound binds to, for example, a double-stranded nucleic acid or a quadruplex nucleic acid, rotary motion of the acceptors changes and the fluorescence property of the compound changes. 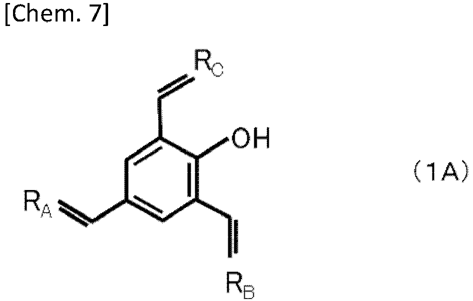 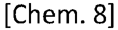 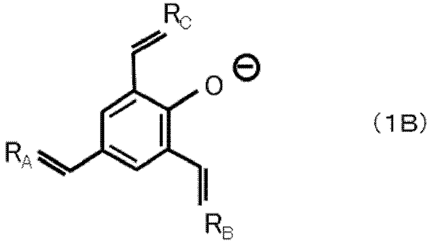 Here,
   R_{A}, R_{B}, and R_{C} each independently have a structure selected from the group consisting of formula (1C), formula (1D), formula (1E), and formula (1F) below (* representing a binding site), 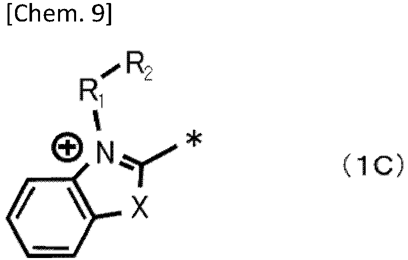 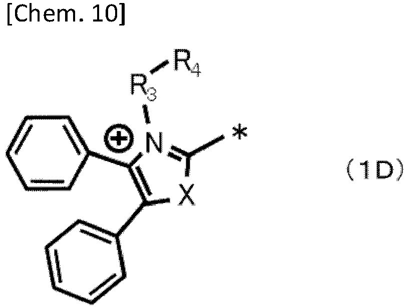 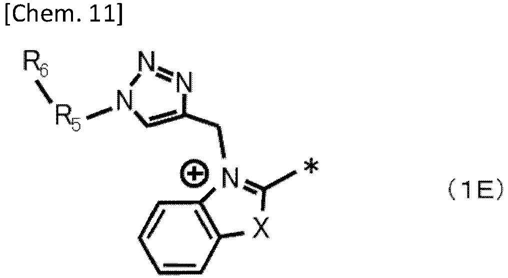 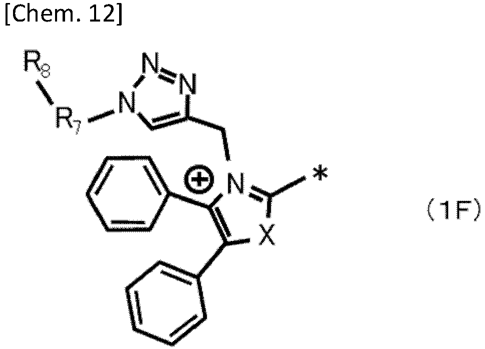
   X is selected from the group consisting of O, S, and Se,
   R₁, R₃, R₅, and R₇ are each independently selected from the group consisting of H, -(CH₂)n-, and -(CH₂CH₂O)n-, n being a natural number of 1 to 100, and
   R₂, R₄, R₆, and R₈ are each independently selected from the group consisting of H, -OH, methyl, an amine, a terminal alkyne, an alkene, an alkyl acid, an amine acid, sulfonate (SO₃⁻), an aryl group or a derivative thereof, and a heterocyclic compound or a derivative thereof.
(7) Preferably, a fluorescent probe according to the embodiment includes the compound according to (6) above.
(8) Preferably, a method according to the embodiment includes using the compound according to (6) above for measurement of a viscosity of a liquid. In the compound according to (6) above, the fluorescence property changes also in accordance with the viscosity of a liquid. Therefore, the compound can be used for measurement of viscosity.
(9) The compound according to the embodiment may be a compound including: a donor; and three acceptors bound to the donor so as to perform rotary motion with respect to the donor, wherein a fluorescence property of the compound changes in accordance with change in the rotary motion of each acceptor.
(10) Preferably, the compound according to (9) above is capable of binding to at least either one of a double-stranded nucleic acid and a quadruplex nucleic acid.
(11) Preferably, the compound according to (9) above is capable of binding to both of a double-stranded nucleic acid and a quadruplex nucleic acid.
(12) Preferably, in the compound according to (11) above, when any two of the three acceptors have bound to a minor groove of the double-stranded nucleic acid, a fluorescence intensity having a first wavelength becomes higher than that when the two acceptors are not bound to the double-stranded nucleic acid, and when the three acceptors have bound to the quadruplex nucleic acid, a fluorescence intensity having a second wavelength different from the first wavelength becomes higher than that when the three acceptors are not bound to the quadruplex nucleic acid.
(13) Preferably, a method according to the embodiment includes using the compound according to any one of (9) to (12) above, for nucleic acid detection.
(14) Preferably, a method according to the embodiment includes using the compound according to any one of (9) to (12) above, for nucleic acid detection in which a double-stranded nucleic acid and a quadruplex nucleic acid are distinguished from each other.
(15) Preferably, a method according to the embodiment includes using the compound according to any one of (9) to (12) above, for measurement of a viscosity of a liquid. In the compound, in accordance with the viscosity of a liquid, rotary motion changes and fluorescence changes. Utilizing this, the compound can be used for measurement of viscosity.

### <2. Examples of overviews of nucleic acid detection method, compound, and fluorescent probe>

### <2.1 Structure of compound>

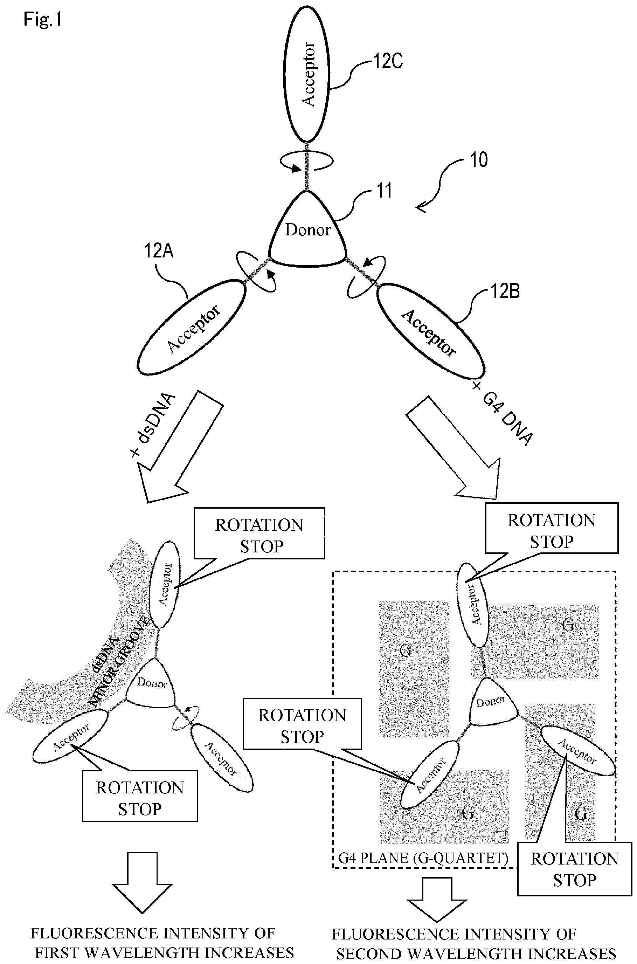

FIG. 1 shows an overview of a compound 10 according to an embodiment, and nucleic acid detection when using the compound 10 as a fluorescent probe. The compound according to the embodiment is suitable for detection of a double-stranded nucleic acid and a quadruplex nucleic acid. The compound 10 as a fluorescent probe is also suitable for measurement of viscosity. The compound 10 has a "donor-three-acceptor (D3A)" structure including one donor 11 (donor moiety) and three acceptors 12A, 12B, 12C (acceptor moieties).

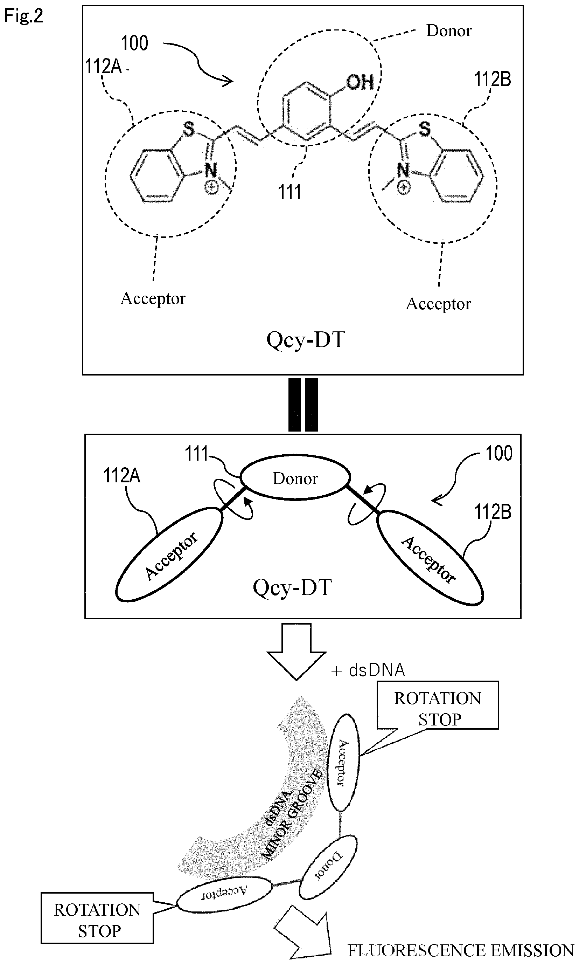

Before describing D3A shown in FIG. 1, QCy-DT, which is one of donor-two-acceptors (D2A), will be described in order to facilitate understanding. FIG. 2 shows a structure of QCy-DT described in PATENT LITERATURE 1 and NON PATENT LITERATURE 1 and an overview of nucleic acid detection when using QCy-DT as a fluorescent probe. QCy-DT is a compound 100 having a D2A structure that includes a donor phenol moiety 111 and two heterocyclic electron acceptors 112A, 112B bound to the donor phenol moiety 111. QCy-DT is one type of a quinone cyanine (Qcy7) fluorescent probe (fluorescent dye). QCy-DT is of a two-leg type. Here, "two-leg type" means a structure that has two acceptors bound to one donor.

The Qcy7 (quinone cyanine) fluorescent dye is one class of a cyanine fluorescent probe (cyanine fluorescent dye). Typically, in Qcy7, two heterocyclic electron acceptors are bound to the donor phenol moiety, and the heterocyclic electron acceptors in Qcy7 are, for example, alkylated quinolines, indolines, or pyridines.

QCy-DT is activated through deprotonation, and functions as a near-infrared fluorescent probe. The donor phenol moiety 111 becomes a phenolate through deprotonation. The phenolate provides an electron to either one (N-alkylated benzothiazole) of the two acceptors 112A, 112B, to trigger internal charge transfer to a nitrogen atom included in the other acceptor of the two acceptors 112A, 112B.

QCy-DT includes benzothiazolium cations (benzothiazole groups) as the acceptors 112A, 112B. Each of the two acceptors 112A, 112B included in QCy-DT can perform rotary motion (twist) about the binding to the donor 111, with the binding serving as a rotation axis. When the rotation of the acceptors 112A, 112B is suppressed or stopped, the fluorescence property of QCy-DT changes. That is, in QCy-DT, the fluorescence property changes in accordance with change in the rotary motion of the acceptors 112A, 112B. The fluorescence property that changes is at least either one of fluorescence wavelength or fluorescence intensity, for example.

When the acceptors 112A, 112B are rotating, the fluorescence is quenched or the intensity thereof decreases. Meanwhile, when the rotation of the acceptors 112A, 112B is suppressed or stopped, the fluorescence intensity at a specific wavelength increases. That is, QCy-DT does not emit fluorescence when the acceptors 112A, 112B are rotating, and emits fluorescence when the rotation of the acceptors 112A, 112B is stopped.

QCy-DT can bind to a minor groove of a double-stranded DNA. When the acceptor 112A, 112B of QCy-DT is not bound to a double-stranded DNA, suppression of the rotation is small, and fluorescence is quenched. However, as shown in FIG. 2, when an activated QCy-DT binds to a minor groove of a double-stranded DNA, rotation of the acceptors 112A, 112B is suppressed or stopped, and fluorescence (near-infrared radiation) having a specific wavelength is caused. Therefore, QCy-DT can be used as a fluorescent probe.

When rotation of both acceptors 112A, 112B is suppressed or stopped due to binding of QCy-DT to a double-stranded DNA, charge transfer is caused between the acceptors 112A, 112B (in this case, one acceptor out of both acceptors 112A, 112B serves as a donor of a charge, and the other acceptor serves as an acceptor of the charge.). Since charge transfer occurs between the acceptors 112A, 112B whose rotation is stopped or suppressed, QCy-DT can emit fluorescence having a long wavelength.

As described above, QCy-DT has a D2A structure, and when the two acceptors 112A, 112B bind to a minor groove of a double-stranded nucleic acid, rotation of the acceptor 112A, 112B is suppressed or stopped, and QCy-DT can emit fluorescence. That is, QCy-DT emits fluorescence upon binding to a minor groove of a double-stranded nucleic acid, whereas, when QCy-DT is not bound thereto, fluorescence becomes weaker than that when QCy-DT is bound thereto. Further, in QCy-DT, since charge transfer is caused between the two acceptors 112A, 112B whose rotation has been suppressed or stopped, QCy-DT can emit fluorescence having a long wavelength.

Further, QCy-DT has sequence selectivity. That is, QCy-DT binds to an AT-rich sequence in a nucleic acid. More specifically, QCy-DT specifically binds to a double-stranded nucleic acid having a sequence of 5'-AAATTT-3'. The bond is a hydrogen bound, for example.

With reference back to FIG. 1, the compound 10 according to the embodiment is of a three-leg type including a first acceptor 12A, a second acceptor 12B, and a third acceptor 12C which are bound to one donor 11. Here, the "three-leg type" means a structure that has three acceptors bound to one donor. The compound 10 according to the embodiment includes the donor 11 and the three acceptors 12A, 12B, 12C bound to the donor 11 so as to perform rotary motion with respect to the donor 11, and the fluorescence property of the compound 10 changes in accordance with change in the rotary motion of each acceptor 12A, 12B, 12B. The compound 10 according to the embodiment can be regarded as QCy-DT of a two-leg type expanded so as to be a three-leg type. The donor 11 included in the compound 10 is composed of phenol, similar to QCy-DT, for example. Each acceptor 12A, 12B, 12C included in the compound 10 is composed of a benzothiazolium cation (benzothiazole group), similar to QCy-DT, for example. Other examples of the acceptors 12A, 12B, 12 will be described later.

The donor phenol moiety 11 having been deprotonated becomes a phenolate. The phenolate provides an electron to any one of the three acceptors 12A, 12B, 12C.

Each of the acceptors 12A, 12B, 12C included in the compound 10 according to the embodiment can perform rotary motion (twist) about the binding to the donor 11, with the binding serving as a rotation axis, similar to the acceptors 112A, 112B included in QCy-DT. The bond with each acceptor 12A, 12B, 12C is a dimethine bond, for example.

Similar to QCy-DT, the compound 10 may be capable of binding to a minor groove of a double-stranded nucleic acid (double-stranded DNA; dsDNA). When two acceptors arbitrarily selected from among the three acceptors 12A, 12B, 12C are focused on, the compound 10 can have a structure similar to that of QCy-DT being D2A. For example, in the compound 10 being D3A, when the donor 11, the first acceptor 12A, and the second acceptor 12B are focused on, the compound 10 can have a structure similar to that of QCy-DT being D2A. Further, also when the donor 11, the second acceptor 12B, and the third acceptor 12C are focused on, the compound 10 can have a structure similar to that of QCy-DT being D2A. Further, also when the donor 11, the third acceptor 12C, and the first acceptor 12A are focused on, the compound 10 can have a structure similar to that of QCy-DT being D2A.

Therefore, similar to QCy-DT, the compound 10 may be capable of binding to a minor groove of a double-stranded nucleic acid such as a double-stranded DNA, by means of two acceptors arbitrarily selected from among the three acceptors 12A, 12B, 12C.

For example, a pair of the first acceptor 12A and the second acceptor 12B in the compound 10 can bind to a minor groove of a double-stranded DNA. In this case, rotation of the first acceptor 12A and the second acceptor 12B bound to the minor groove is suppressed or stopped. When the rotation of the two acceptors 12A, 12B is suppressed or stopped, fluorescence (near-infrared radiation) having a specific wavelength is caused, similar to QCy-DT. However, the third acceptor 12C not bound to the minor groove is rotatable.

A pair of the second acceptor 12B and the third acceptor 12C in the compound 10 can bind to a minor groove of a double-stranded DNA. In this case, rotation of the second acceptor 12B and the third acceptor 12C bound to the minor groove is suppressed or stopped. When the rotation of the two acceptors 12B, 12C is suppressed or stopped, fluorescence (near-infrared radiation) having a specific wavelength is caused, similar to QCy-DT. However, the first acceptor 12A not bound to the minor groove is rotatable.

A pair of the third acceptor 12C and the first acceptor 12A in the compound 10 can bind to a minor groove of a double-stranded DNA. In this case, rotation of the third acceptor 12C and the first acceptor 12A bound to the minor groove is suppressed or stopped. When the rotation of the two acceptors 12C, 12A is suppressed or stopped, fluorescence (near-infrared radiation) having a specific wavelength is caused, similar to QCy-DT. However, the second acceptor 12B not bound to the minor groove is rotatable.

As described above, in the compound 10 which is of a three-leg type, the fluorescence property changes in accordance with change in rotary motion of the acceptors 12A, 12B, 12C, similar to QCy-DT. Therefore, preferably, the compound 10 can be used as a fluorescent probe (fluorescent dye) for detection of a double-stranded nucleic acid. That is, the compound 10 may emit fluorescence upon binding to a minor groove of a double-stranded nucleic acid, whereas, when the compound 10 is not bound thereto, the fluorescence intensity may become lower than that when the compound 10 is bound thereto. It should be noted that, in the compound 10 as well, charge transfer is caused between acceptors whose rotation has been stopped or suppressed, and thus, the compound 10 can emit fluorescence having a long wavelength. Further, the compound 10 may have sequence selectivity, similar to QCy-DT. That is, the compound 10 may bind to an AT-rich sequence in a nucleic acid. More specifically, the compound 10 may specifically bind to a double-stranded nucleic acid having a sequence of 5'-AAATTT-3'.

The compound 10, which is of a three-leg type, can bind not only to a double-stranded nucleic acid but also a quadruplex nucleic acid. Since the compound 10 of the three-leg type includes three acceptors 12A, 12B, 12C, the compound 10 has a molecular structure in a planar shape as a whole, when compared with the compound 100 of the two-leg type. Being in a planar shape, the compound 10 can bind to a G4 plane (G-Quartet) of a quadruplex nucleic acid.

Here, the quadruplex nucleic acid is also referred to as a guanine quadruplex (G-quadruplex: G4). The G-quadruplex is formed by a DNA or RNA that is rich in guanine. The G-quadruplex has a structure in which plane structures (G4 planes) each referred to as a G-Quartet formed by four guanine bases are stacked. The structure of the G-quadruplex (G4) may be any of a parallel type, an antiparallel type, and a hybrid type. With any structure thereamong, the G-quadruplex has a G4 plane formed by four guanine bases.

Being in a planar shape, the compound 10 has a characteristic of easily binding to the G4 plane (G-Quartet), which is also a plane. That is, the compound 10 in a planar shape can bind to the G4 plane so as to overlap the G4 plane in a planar manner. The bond between the compound 10 and the quadruplex nucleic acid may be a hydrophobic bond, or may be realized as a stack due to a binding force generated between planes through π-π stacking interaction.

Since the compound 10 overlaps the G4 plane in a planar manner, all of the three acceptors 12A, 12B, 12C bind to the quadruplex nucleic acid. Therefore, when the compound 10 binds to the quadruplex nucleic acid, rotation of all of the three acceptors 12A, 12B, 12C is suppressed or stopped. When the rotation of the three acceptors 12A, 12B, 12C is suppressed or stopped, fluorescence (near-infrared radiation) is caused. Thus, the compound 10 of the three-leg type can be used as a fluorescent probe (fluorescent dye) for detection of a quadruplex nucleic acid. That is, the compound 10 emits fluorescence upon binding to a quadruplex nucleic acid, whereas, when the compound 10 is not bound thereto, the fluorescence intensity becomes lower than that when the compound 10 is bound thereto.

When rotation of all of the three acceptors 12A, 12B, 12C is suppressed or stopped, fluorescence (near-infrared radiation) having a wavelength different from that when rotation of two acceptors is suppressed or stopped, is caused. That is, the wavelength at which the fluorescence intensity increases when the compound 10 binds to a double-stranded nucleic acid, and the wavelength at which the fluorescence intensity increases when the compound 10 binds to a quadruplex nucleic acid are different from each other. Therefore, when the compound 10 is used to detect a nucleic acid, whether the detected nucleic acid is a double-stranded nucleic acid or a quadruplex nucleic acid can be distinguished by the color of the fluorescence (the wavelength of the fluorescence), from the compound 10, whose fluorescence intensity increases.

As described above, the compound 10 emits fluorescence when rotation of a part or all of the three acceptors 12A, 12B, 12C is suppressed or stopped. Fluorescence occurs not only when binding to a double-stranded nucleic acid or a quadruplex nucleic acid, but also when rotation of a part or all of the three acceptors 12A, 12B, 12C is suppressed or stopped due to another factor. The other factor is the viscosity of a liquid in which the compound 10 is present, for example. When the viscosity is high, rotation of the acceptors 12A, 12B, 12C is suppressed.

### <2.2 Example of compound>

The compound 10 is represented by general formula (1A) or general formula (1B) described above, for example. The compound 10 is of a three-leg type and is used as a novel quinone cyanine fluorescent probe (quinone cyanine fluorescent dye).

The compound 10 represented by general formula (1A) has phenol as the donor 11 (donor moiety), and three acceptors (acceptor moieties: R_{A}, R_{B}, and R_{C} in general formula (1A), (1B)) are bound to the phenol. The compound 10 represented by general formula (1B) is one (activated compound 10) obtained by deprotonating the compound 10 represented by general formula (1A), and the phenol in general formula (1A) has been changed to a phenolate. The activated compound 10 functions as a near-infrared fluorescent probe.

R_{A}, R_{B}, and R_{C} (acceptors) in general formula (1A), (1B) each independently have a structure selected from the group consisting of formula (1C), formula (1D), formula (1E), and formula (1F) described above. With respect to R_{A}, R_{B}, and R_{C}, all of them may have a structure represented by formula (1C), all of them may have a structure represented by formula (1D), all of them may have a structure represented by formula (1E), or all of them may have a structure represented by formula (1F). All of R_{A}, R_{B}, and R_{C} in general formula (1A), (1B) may have structures different from each other. The structure represented by formula (1D) is more planar than the structure represented by formula (1C), and is advantageous for binding to a quadruplex nucleic acid. The structure represented by formula (1E) is more planar than the structure represented by formula (1C), and is advantageous for binding to a quadruplex nucleic acid. The structure represented by formula (1F) is more planar than the structure represented by formula (1D), and is advantageous for binding to a quadruplex nucleic acid.

R_{A} may have the structure represented by formula (1C), R_{B} may have the structure represented by formula (1C), and R_{C} may have a structure selected from the group consisting of formula (1C), formula (1D), formula (1E), and formula (1F).

R_{A} may have the structure represented by formula (1C), R_{B} may have the structure represented by formula (1D), and R_{C} may have a structure selected from the group consisting of formula (1C), formula (1D), formula (1E), and formula (1F).

R_{A} may have the structure represented by formula (1C), R_{B} may have the structure represented by formula (1E), and R_{C} may have a structure selected from the group consisting of formula (1C), formula (1D), formula (1E), and formula (1F).

R_{A} may have the structure represented by formula (1C), R_{B} may have the structure represented by formula (1F), and R_{C} may have a structure selected from the group consisting of formula (1C), formula (1D), formula (1E), and formula (1F).

R_{A} may have the structure represented by formula (1D), R_{B} may have the structure represented by formula (1C), and R_{C} may have a structure selected from the group consisting of formula (1C), formula (1D), formula (1E), and formula (1F).

R_{A} may have the structure represented by formula (1D), R_{B} may have the structure represented by formula (1D), and R_{C} may have a structure selected from the group consisting of formula (1C), formula (1D), formula (1E), and formula (1F).

R_{A} may have the structure represented by formula (1D), R_{B} may have the structure represented by formula (1E), and R_{C} may have a structure selected from the group consisting of formula (1C), formula (1D), formula (1E), and formula (1F).

R_{A} may have the structure represented by formula (1D), R_{B} may have the structure represented by formula (1F), and R_{C} may have a structure selected from the group consisting of formula (1C), formula (1D), formula (1E), and formula (1F).

R_{A} may have the structure represented by formula (1E), R_{B} may have the structure represented by formula (1C), and R_{C} may have a structure selected from the group consisting of formula (1C), formula (1D), formula (1E), and formula (1F).

R_{A} may have the structure represented by formula (1E), R_{B} may have the structure represented by formula (1D), and R_{C} may have a structure selected from the group consisting of formula (1C), formula (1D), formula (1E), and formula (1F).

R_{A} may have the structure represented by formula (1E), R_{B} may have the structure represented by formula (1E), and R_{C} may have a structure selected from the group consisting of formula (1C), formula (1D), formula (1E), and formula (1F).

R_{A} may have the structure represented by formula (1E), R_{B} may have the structure represented by formula (1F), and R_{C} may have a structure selected from the group consisting of formula (1C), formula (1D), formula (1E), and formula (1F).

R_{A} may have the structure represented by formula (1F), R_{B} may have the structure represented by formula (1C), and R_{C} may have a structure selected from the group consisting of formula (1C), formula (1D), formula (1E), and formula (1F).

R_{A} may have the structure represented by formula (1F), R_{B} may have the structure represented by formula (1D), and R_{C} may have a structure selected from the group consisting of formula (1C), formula (1D), formula (1E), and formula (1F).

R_{A} may have the structure represented by formula (1F), R_{B} may have the structure represented by formula (1E), and R_{C} may have a structure selected from the group consisting of formula (1C), formula (1D), formula (1E), and formula (1F).

R_{A} may have the structure represented by formula (1F), R_{B} may have the structure represented by formula (1F), and R_{C} may have a structure selected from the group consisting of formula (1C), formula (1D), formula (1E), and formula (1F).

Examples of general acceptors are described in PATENT LITERATURE 1, which discloses QCy-DT. Therefore, as the acceptors included in the compound 10, acceptors similar to those described in PATENT LITERATURE 1 can be adopted. In selection of the acceptors, NON PATENT LITERATURE 1 and NON PATENT LITERATURE 2 may be referred to. In formula (1C), (1D), (1E), or (1F), X is preferably selected from the group consisting of O, S, and Se, similar to PATENT LITERATURE 1. When X is an element selected from the group consisting of O, S, and Se, each acceptor can perform rotary motion. More preferably, X is S.

Similar to PATENT LITERATURE 1, R₁ in formula (1D), (1D), (1E), or (1E) is preferably H or -(CH₂)n-. R₁ may be -(CH₂CH₂O)n-. That is, R₁ is preferably selected from the group consisting of H, -(CH₂)n-, and -(CH₂CH₂O)n-. Here, n is a natural number of 1 to 100. When n is changed, the rotation speed of the acceptor can be controlled.

Preferably, R₃ is H or -(CH₂)n-. R₃ may be -(CH₂CH₂O)n-. That is, R₃ is preferably selected from the group consisting of H, -(CH₂)n-, and -(CH₂CH₂O)n-. Here, n is a natural number of 1 to 100. When n is changed, the rotation speed of the acceptor can be controlled.

Preferably, R₅ is H or -(CH₂)n-. R₅ may be -(CH₂CH₂O)n-. That is, R₅ is preferably selected from the group consisting of H, -(CH₂)n-, and -(CH₂CH₂O)n-. Here, n is a natural number of 1 to 100. When n is changed, the rotation speed of the acceptor can be controlled.

Preferably, R₇ is H or -(CH₂)n-. R₅ may be -(CH₂CH₂O)n-. That is, R₅ is preferably selected from the group consisting of H, -(CH₂)n-, and -(CH₂CH₂O)n-. Here, n is a natural number of 1 to 100. When n is changed, the rotation speed of the acceptor can be controlled.

-R₁-R₂ may be -(CH₂)n-H, an oligoethylene glycol chain (-(CH₂CH₂O)n-H), or (-(CH₂CH₂O)n-CH₃), and in this case, the compound 10 is suitable as a viscosity probe for viscosity measurement. When -R₁-R₂ is -(CH₂)n-H, n is preferably a natural number of 1 to 18. When -R₁-R₂ is -(CH₂CH₂O)n-H or -(CH₂CH₂O)n-CH₃, n is preferably a natural number of 1 to 100, more preferably a natural number of 1 to 36, and further preferably a natural number of 1 to 6.

-R₃-R₄ may be -(CH₂)n-H, an oligoethylene glycol chain (-(CH₂CH₂O)n-H), or (-(CH₂CH₂O)n-CH₃), and in this case, the compound 10 is suitable as a viscosity probe for viscosity measurement. When -R₃-R₄ is -(CH₂)n-H, n is preferably a natural number of 1 to 18. When -R₃-R₄ is -(CH₂CH₂O)n-H or -(CH₂CH₂O)n-CH₃, n is preferably a natural number of 1 to 100, more preferably a natural number of 1 to 36, and further preferably a natural number of 1 to 6.

-R₅-R₆ may be -(CH₂)n-H, an oligoethylene glycol chain (-(CH₂CH₂O)n-H), or (-(CH₂CH₂O)n-CH₃), and in this case, the compound 10 is suitable as a viscosity probe for viscosity measurement. When -R₅-R₆ is -(CH₂)n-H, n is preferably a natural number of 1 to 18. When -R₅-R₆ is -(CH₂CH₂O)n-H or -(CH₂CH₂O)n-CH₃, n is preferably a natural number of 1 to 100, more preferably a natural number of 1 to 36, and further preferably a natural number of 1 to 6.

-R₇-R₈ may be -(CH₂)n-H, an oligoethylene glycol chain (-(CH₂CH₂O)n-H), or (-(CH₂CH₂O)n-CH₃), and in this case, the compound 10 is suitable as a viscosity probe for viscosity measurement. When -R₇-R₈ is -(CH₂)n-H, n is preferably a natural number of 1 to 18. When -R₇-R₈ is -(CH₂CH₂O)n-H or -(CH₂CH₂O)n-CH₃, n is preferably a natural number of 1 to 100, more preferably a natural number of 1 to 36, and further preferably a natural number of 1 to 6.

Similar to PATENT LITERATURE 1, R₂ is also preferably selected from the group consisting of H, -OH, methyl, an amine, a terminal alkyne, an alkene, an alkyl acid, an amine acid, and sulfonate (SO₃⁻). R₂ may be an aryl group such as phenyl (C₆H₅), naphthyl, or pyrene, or a derivative thereof. R₂ may be a heterocyclic compound such as thiophene, pyridyl, furan, imidazole, triazole, carbazole, or coumarin, or a derivative thereof. That is, R₂ is preferably selected from the group consisting of H, - OH, methyl, an amine, a terminal alkyne, an alkene, an alkyl acid, an amine acid, sulfonate (SO₃⁻), an aryl group or a derivative thereof, and a heterocyclic compound or a derivative thereof.

R₄ may also be an aryl group such as phenyl (C₆H₅), naphthyl, or pyrene, or a derivative thereof. R₄ may be a heterocyclic compound such as thiophene, pyridyl, furan, imidazole, triazole, carbazole, or coumarin, or a derivative thereof. That is, R₄ is preferably selected from the group consisting of H, -OH, methyl, an amine, a terminal alkyne, an alkene, an alkyl acid, an amine acid, sulfonate (SO₃⁻), an aryl group or a derivative thereof, and a heterocyclic compound or a derivative thereof.

R₆ may also be an aryl group such as phenyl (C₆H₅), naphthyl, or pyrene, or a derivative thereof. R₆ may be a heterocyclic compound such as thiophene, pyridyl, furan, imidazole, triazole, carbazole, or coumarin, or a derivative thereof. That is, R₆ is preferably selected from the group consisting of H, -OH, methyl, an amine, a terminal alkyne, an alkene, an alkyl acid, an amine acid, sulfonate (SO₃⁻), an aryl group or a derivative thereof, and a heterocyclic compound or a derivative thereof.

R₈ may also be an aryl group such as phenyl (C₆H₅), naphthyl, or pyrene, or a derivative thereof. R₄ may be a heterocyclic compound such as thiophene, pyridyl, furan, imidazole, triazole, carbazole, or coumarin, or a derivative thereof. That is, R₄ is preferably selected from the group consisting of H, -OH, methyl, an amine, a terminal alkyne, an alkene, an alkyl acid, an amine acid, sulfonate (SO₃⁻), an aryl group or a derivative thereof, and a heterocyclic compound or a derivative thereof.

Preferably, the compound 10 represented by general formula (1A) is a compound 10 represented by general formula (2A) below. The compound 10 represented by general formula (2A) has adopted the structure represented by formula (1C) as all of R_{A}, R_{B}, and R_{C} in general formula (1A).

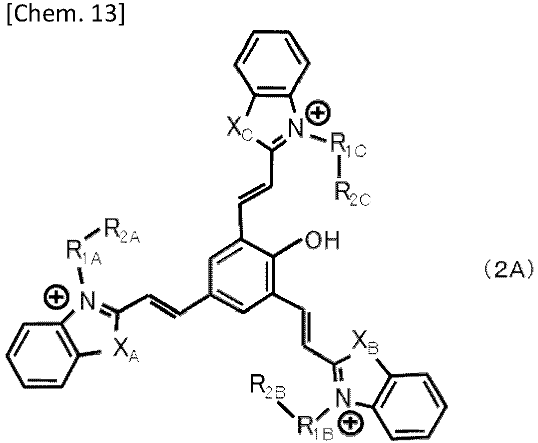

Preferably, the compound 10 represented by general formula (1B) is a compound 10 represented by general formula (2B) below. The compound 10 represented by general formula (2B) has adopted the structure represented by formula (1C) as all of R_{A}, R_{B}, and R_{C} in general formula (1B).

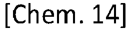

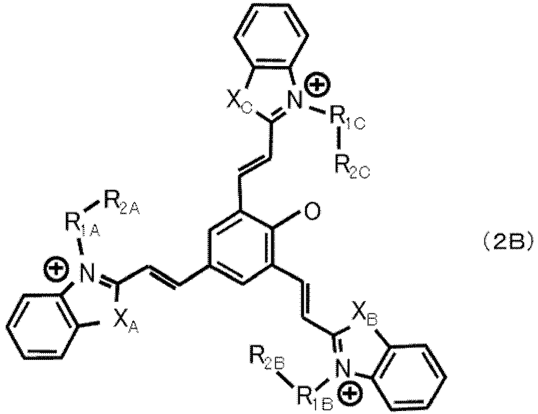

The compound 10 represented by each of general formulae (2A) and (2B) includes a donor and three acceptors bound to the donor so as to perform rotary motion with respect to the donor, and the fluorescence property of the compound 10 changes in accordance with change in the rotary motion of each acceptor.

In general formulae (2A) and (2B), it is preferable that X_{A}, X_{B}, and X_{C} are each independently selected from the group consisting of O, S, and Se. Preferably, X_{A}, X_{B}, and X_{C} are each independently S. X_{A}, X_{B}, and X_{C} may all be S.

Preferably, R_{1A}, R_{1B}, and R_{1C} are each independently similar to R₁ described above. That is, preferably, R_{1A}, R_{1B}, and R_{1C} are each independently selected from the group consisting of H, -(CH₂)n-, and -(CH₂CH₂O)n-. Here, n is a natural number of 1 to 100. R_{1A}, R_{1B}, and R_{1C} may all be the same.

Preferably, R_{2A}, R_{2B}, and R_{2C} are each independently similar to R₂ described above. That is, preferably, R_{2A}, R_{2B}, and R_{2C} are each independently selected from the group consisting of H, -OH, methyl, an amine, a terminal alkyne, an alkene, an alkyl acid, an amine acid, sulfonate (SO₃⁻), an aryl group or a derivative thereof, and a heterocyclic compound or a derivative thereof. R_{2A}, R_{2B}, and R_{2C} may all be the same.

-R_{1A}-R_{2A}, -R_{1B}-R_{2B}, and -R_{1C}-R_{2C} may each be independently -(CH₂)n-H, an oligoethylene glycol chain (-(CH₂CH₂O)n-H), or (-(CH₂CH₂O)n-CH₃).

The compound 10 represented by each of general formulae (2A) and (2B) is suitable for detection of at least either one of a double-stranded nucleic acid and a quadruplex nucleic acid. In order to ensure binding performance to a double-stranded nucleic acid, the acceptor moieties 12A, 12B, 12C of the compound 10 preferably have structures similar to those of the acceptor moieties of QCy-DT. Meanwhile, planarity of the structure contributes to binding to a quadruplex nucleic acid. Therefore, the structures of the acceptor moieties 12A, 12B, 12C of the compound 10 may have low similarities to those of the acceptor moieties of QCy-DT. For example, when a large functional group is introduced to R₂ of all of the three acceptor moieties, the binding performance to a quadruplex nucleic acid is improved due to increase in the stacking interaction through expansion of the planar structure, although the binding performance to a double-stranded nucleic acid may be decreased. In this case, the compound 10 is suitable as a quadruplex nucleic acidselective fluorescent probe.

When, out of R_{2A}, R_{2B}, and R_{2C} included in the three acceptors of the compound 10, any two are set to H, and the remaining one is set to a functional group having a high planarity, the QCy-DT structure is maintained by the two acceptors and the donor included in the compound 10. In this case, a double-stranded nucleic acid and a quadruplex nucleic acid can be detected by fluorescences having different wavelengths.

Preferably, the compound 10 represented by general formula (2A) is a compound 10 represented by general formula (2C) below. The compound 10 represented by general formula (2C) includes three benzothiazolium cations via dimethine bonds with phenol as the center. In the compound 10 represented by general formula (2C), R_{1A}, R_{1B}, and R_{1C} in general formula (2A) are CH₂, and R_{2A}, R_{2B}, and R_{2C} therein are H.

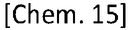

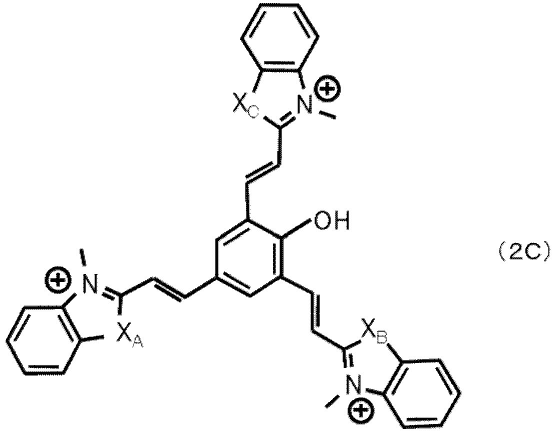

Preferably, the compound 10 represented by general formula (2B) is a compound 10 represented by general formula (2D) below. In the compound 10 represented by general formula (2D), R₁ in general formula (2B) is CH₂, and R₂ therein is H.

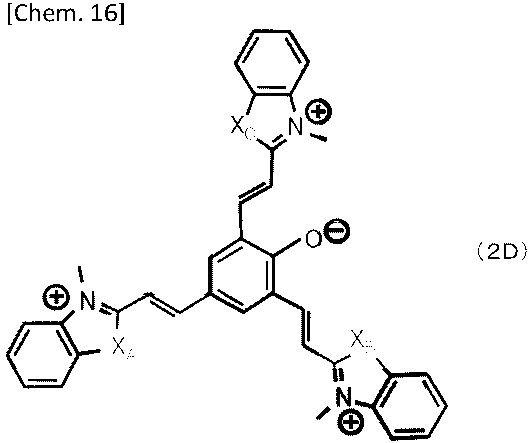

In general formulae (2C) and (2D), it is preferable that X_{A}, X_{B}, and X_{C} are each independently selected from the group consisting of O, S, and Se. Preferably, X_{A}, X_{B}, and X_{C} are each independently S. X_{A}, X_{B}, and X_{C} may all be S.

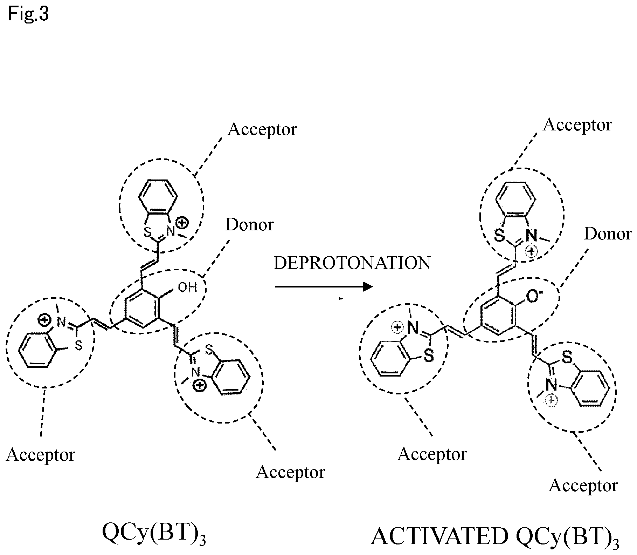

As shown in FIG. 3, the compound 10 in which X_{A}, X_{B}, and X_{C} in general formula (2C) are all S may be referred to as "QCy(BT)₃". The compound 10 in which X_{A}, X_{B}, and X_{C} in general formula (2D) are all S may be referred to as "activated QCy(BT)₃". The compound 10 represented by general formula (2D) is one (activated compound 10) obtained by deprotonating the compound 10 represented by general formula (2C).

Preferably, the compound 10 represented by general formula (1A) is a compound 10 represented by general formula (3A) below. The compound 10 represented by general formula (3A) has adopted the structure represented by formula (1D) as all of R_{A}, R_{B}, and R_{C} in general formula (1A). The compound 10 represented by general formula (3A) is more planar than the compound 10 represented by general formula (2A), and is advantageous for binding to a quadruplex nucleic acid.

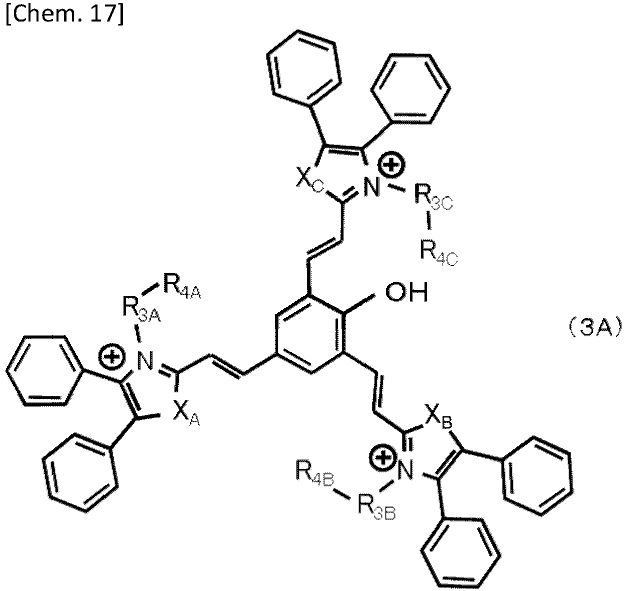

Preferably, the compound 10 represented by general formula (1B) is a compound 10 represented by general formula (3B) below. The compound 10 represented by general formula (3B) has adopted the structure represented by formula (1D) as all of R_{A}, R_{B}, and R_{C} in general formula (1B). The compound 10 represented by general formula (3B) is more planar than the compound 10 represented by general formula (2B), and is advantageous for binding to a quadruplex nucleic acid.

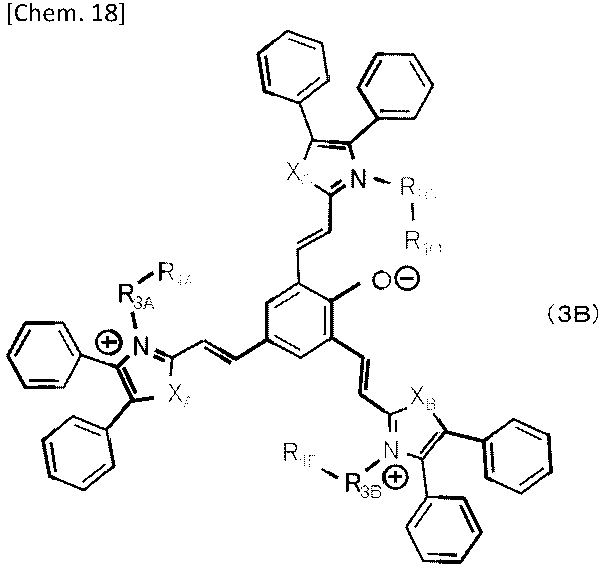

Similar to the compound 10 represented by each of general formulae (2A) and (2B), the compound 10 represented by each of general formulae (3A) and (3B) includes a donor and three acceptors bound to the donor so as to perform rotary motion with respect to the donor, and the fluorescence property of the compound 10 changes in accordance with change in the rotary motion of each acceptor.

In general formulae (3A) and (3B), it is preferable that X_{A}, X_{B}, and X_{C} are each independently selected from the group consisting of O, S, and Se. Preferably, X_{A}, X_{B}, and X_{C} are each independently S. X_{A}, X_{B}, and X_{C} may all be S.

Preferably, R_{3A}, R_{3B}, and R_{3C} are each independently similar to R₁ or R₃ described above. That is, preferably, R_{3A}, R_{3B}, and R_{3C} are each independently selected from the group consisting of H, -(CH2)n-, and -(CH₂CH₂0)n-. Here, n is a natural number of 1 to 100. R_{3A}, R_{3B}, and R_{3C} may all be the same.

Preferably, R_{4A}, R_{4B}, and R_{4C} are each independently similar to R₂ or R₄ described above. That is, preferably, R_{4A}, R_{4B}, and R_{4C} are each independently selected from the group consisting of H, -OH, methyl, an amine, a terminal alkyne, an alkene, an alkyl acid, an amine acid, sulfonate (SO₃⁻), an aryl group or a derivative thereof, and a heterocyclic compound or a derivative thereof. R_{4A}, R_{4B}, and R_{4C} may all be the same.

-R_{3A}-R_{4A}, -R_{3B}-R_{4B}, and -R_{3C}-R_{4C} may each be independently -(CH₂)n-H, an oligoethylene glycol chain (-(CH₂CH₂O)n-H), or (-(CH₂CH₂O)n-CH₃).

Preferably, in general formula (3A) or (3B), R_{3A}, R_{3B}, and R_{3C} are CH₂ and R_{4A}, R_{4B}, and R_{4C} are H.

The compound 10 represented by general formula (3B) is one (activated compound 10) obtained by deprotonating the compound 10 represented by general formula (3A).

The compound 10 represented by each of general formulae (3A) and (3B) is also suitable for detection of at least either one of a double-stranded nucleic acid and a quadruplex nucleic acid. The compound 10 represented by each of general formulae (3A) and (3B) has large planarity, and thus, is advantageous for binding to a quadruplex nucleic acid.

### <2.3 Production method of compound>

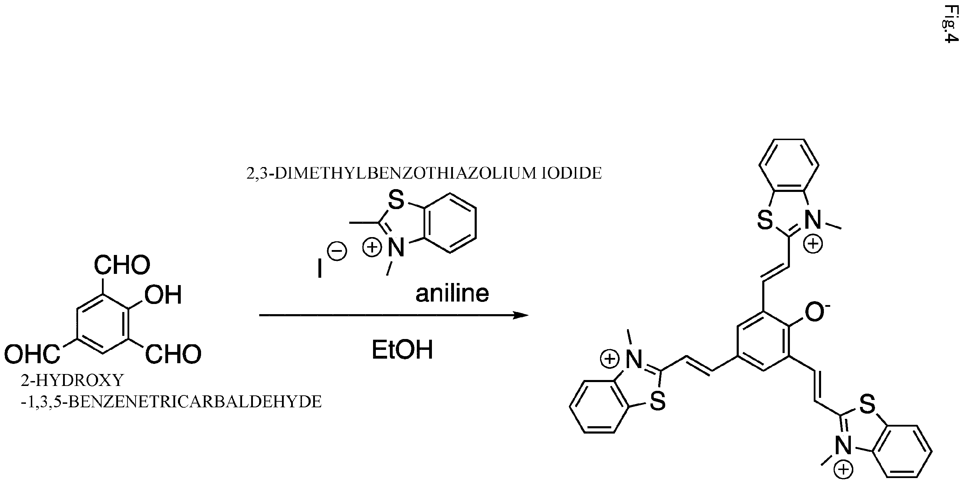

FIG. 4 shows a production method of an activated QCy(DT)₃ (compound 10 in which X_{A}, X_{B}, and X_{C} in formula (2D) are all S). As shown in FIG. 4, 2,3-dimethylbenzothiazolium iodide (50 mg, 172 µmol) and 2-hydroxy-1,3,5-benzenetricarbaldehyde (9 mg, 5 µmol) were stirred in ethanol (5 mL) at 95°C for 10 minutes, and then, aniline (14 µL, 152 mmol) was added thereto. 2,3-dimethylbenzothiazolium iodide is a starting material (precursor) corresponding to the acceptor moieties 12A, 12B, 12C, and 2-hydroxy-1,3,5-benzenetricarbaldehyde is a starting material (precursor) corresponding to the donor moiety 11. The counter anion for 2,3-dimethylbenzothiazolium iodide is not limited to I⁻, and may be Cl^{-,} Br⁻, or a tosyl anion.

The mixture was subjected to heating reflux under an N₂ atmosphere for 6 hours, then, the solvent was removed, and the residue was purified with a reverse phase column (SepPak, eluent: acetonitrile-water (0.1% TFA)). The purified matter was freeze-dried, whereby a compound 10 was obtained. The obtained compound 10 was a deep purple solid. The amount of the obtained compound 10 was 8.3 mg, 9.9 µmol, and the yield was 20%. The obtained compound 10 corresponds to the compound in which X_{A}, X_{B}, and X_{C} in formula (2D) are all S.

Analysis data of the obtained compound 10 is as follows.
1H-NMR: 1H-NMR (400 MHz, DMSO-D6) δ 8.40 (d, J = 15 Hz, 2H), 8.30 (s, 2H), 8.24 (d, J = 7.6 Hz, 2H), 8.18 (d, J = 8.4 Hz, 1H), 8.12 (d, J = 8.8 Hz, 2H), 8.02 (d, J = 8.0 Hz, 1H), 8.00 (d, J = 16 Hz, 2H), 7.86 (d, J = 15 Hz, 1H), 7.74 (t, J = 8.0 Hz, 2H), 7.68 (t, J = 8.2 Hz, 1H), 7.63 (t, J = 7.4 Hz, 2H), 7.56 (t, J = 7.8 Hz, 1H), 7.51 (d, J = 16 Hz, 1H), 4.23 (s, 3H), 4.22 (s, 6H)
13C-NMR: 13C-NMR (101 MHz, DMSO-D6) δ 178.4, 172.2, 170.4, 158.3, 158.0, 149.2, 146.9, 141.7, 141.7, 140.2, 129.0, 128.8, 127.7, 127.3, 126.9, 126.4, 126.2, 123.8, 123.6, 118.4, 118.4, 116.1, 115.6, 115.5, 109.9, 105.6, 35.7, 35.4
ESI-MS: 307.92 (found), 307.57 (calcd. for C36H29N3OS3 2+)

In order to obtain a three-leg type (D3A) like the compound 10 according to the embodiment, it is preferable to use a substance having three aldehyde groups (-CHO) corresponding to the three-leg type, such as 2-hydroxy-1,3,5-benzenetricarbaldehyde described above, as a starting material (a starting material (precursor) corresponding to the donor moiety), for example. When the starting material has two aldehyde groups, a two-leg type (D2A) such as QCy-DT is obtained.

As a production method of the compound 10 other than the production method shown in FIG. 4, a substance suitable for the three-leg type may be adopted as the starting material corresponding to the donor moiety, while being based on the production method of the compound (e.g., QCy-DT) of the two-leg type (D2A). The starting material (precursor) corresponding to the acceptor moieties is not limited to 2,3-dimethylbenzothiazolium iodide, either.

In general, when a molecule has an active methylene group (corresponding to the methyl group at position 2 of 2,3-dimethylbenzothiazolium iodide), if the molecule is condensed with an aldehyde through Knoevenagel condensation, an olefin structure (double bond) can be formed. The production method described above uses this. Therefore, as the precursor of the acceptor moieties according to the embodiment, 2-methyl-3-(R₁R₂)-benzothiazolium iodide (the counter anion is not limited to I-, and may be Cl⁻, Br⁻, or a tosyl anion) or 4,5-diphenyl-2-methyl-3-(R₁R₂)-thiazolium iodide (the counter anion is not limited to I⁻, and may be Cl⁻, Br⁻, or a tosyl anion) may be used. In this case, similar to the production method described above, the structure of the three-leg type (D3A) according to the embodiment can be obtained through Knoevenagel condensation with 2-hydroxy-1,3,5-benzenetricarbaldehyde.

2,3-dimethylbenzothiazolium iodide can be synthesized by causing 2-methylbenzothiazole as a raw material to react with methyl iodide (CH₃I). 2-methyl-3-(R₁R₂)-benzothiazolium iodide (or bromide) can be synthesized by causing 2-methylbenzothiazole as a raw material to react with a compound represented by general formula R₂CH₂I (or R₂CH₂Br).

When the compound 10 of the three-leg type (D3A) according to the embodiment is to be produced, the molecules (the precursor for the acceptors) to serve as the source of the acceptors may be of one type, two types, or three types. For example, to a trialdehyde to serve as a donor, a 3 molar equivalent or more of a mixture of two or more types of molecules (the precursor of the acceptors) to serve as the source of the acceptors is added to react, whereby a compound 10 can be generated. In this case, the generated product is a mixture of compounds 10 having different structures.

When a first molecule, a second molecule, and a third molecule to serve as the source of the acceptors are sequentially caused to react with a trialdehyde to serve as the donor, a desired molecule can be bound to a desired position in the compound 10.

### <3. Experiment example>

### <3.1 First experiment: fluorescence response to solvent viscosity>

In a first experiment, the compound 10 (6 µM) synthesized as shown in "2.3 Production method of compound" and FIG. 4 was dissolved in a solvent, and a fluorescence spectrum and an excitation spectrum were measured. The solvent was made by mixing glycerol into ultrapure water. As the solvent, 11 types of solvents in which the solvent viscosity was changed were prepared by changing the mixing ratio of glycerol. The mixing ratio (the proportion of glycerol to the entire solvent) of glycerol was set to 0 wt%, 10 wt%, 20 wt%, 30 wt%, 40 wt%, 50 wt%, 60 wt%, 70 wt%, 80 wt%, 90 wt%, and 100 wt%. The greater the mixing ratio of glycerol is, the higher the viscosity of the solvent becomes.

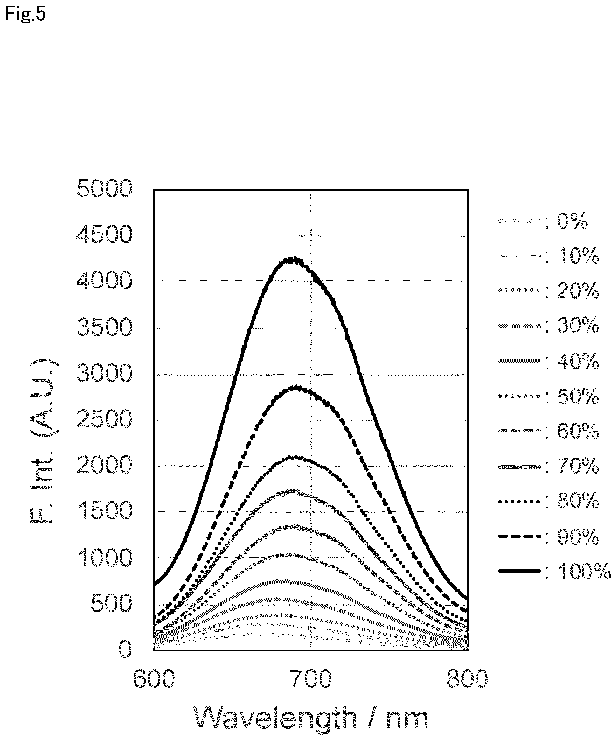

FIG. 5 shows a measurement result of the fluorescence spectrum (excitation wavelength: 540 nm) in the first experiment. In FIG. 5, the vertical axis represents fluorescence intensity, and the horizontal axis represents wavelength. In FIG. 5, the fluorescence intensity in each of the 11 types of solvents is shown. As shown in FIG. 5, it was found that, when the viscosity of the solvent having the compound 10 dissolved therein is increased, the fluorescence intensity (wavelength: 680 nm) increases. When the viscosity of the solvent in which the compound 10 is present is high, rotation of the acceptors 12A, 12B, 12C is suppressed or stopped, and the fluorescence intensity increases. The higher the viscosity is, the higher the fluorescence intensity becomes. The fluorescence intensity became high at a wavelength of 680 nm. Meanwhile, when the viscosity of the solvent in which the compound 10 is present is low, rotation of the acceptors 12A, 12B, 12C is not suppressed, and thus, the fluorescence intensity gradually becomes low. When the viscosity has become sufficiently low, fluorescence is quenched.

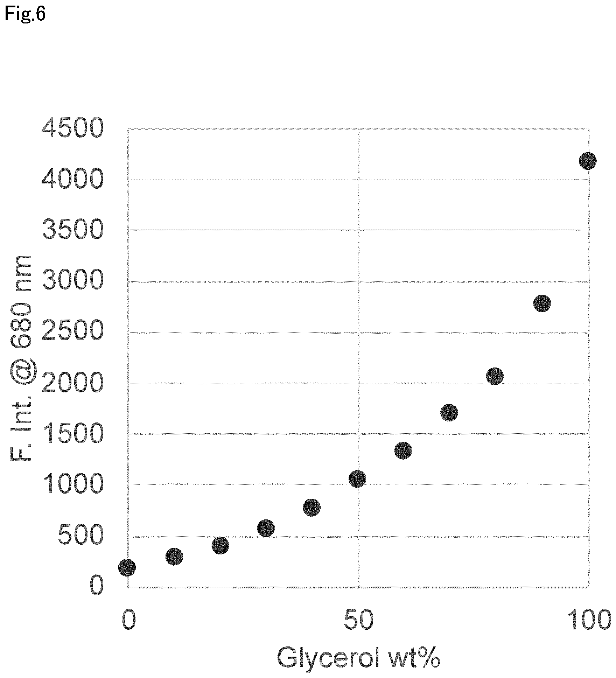

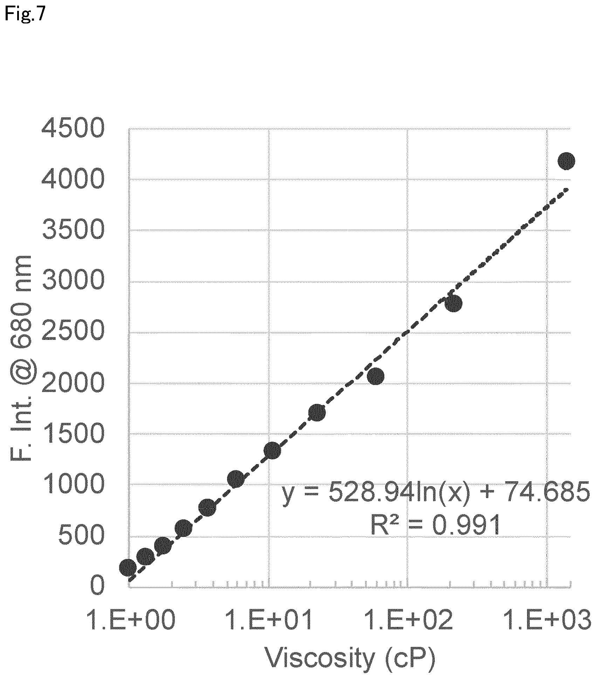

FIG. 6 and FIG. 7 each show correlation between the fluorescence intensity and the solvent viscosity. In FIG. 6, the vertical axis represents fluorescence intensity at 680 nm, and the horizontal axis represents mixing ratio of glycerol. FIG. 7 is obtained by representing the horizontal axis in FIG. 6 in terms of viscosity (logarithm) of the solvent. As shown in FIG. 7, the fluorescence intensity linearly changes with respect to the logarithm of the solvent viscosity. Therefore, when the compound 10 is used, the viscosity (solvent viscosity) of the liquid in which the compound 10 is present can be measured by using the fluorescence intensity at a specific wavelength (e.g., 680 nm) emitted by the compound 10, as an index.

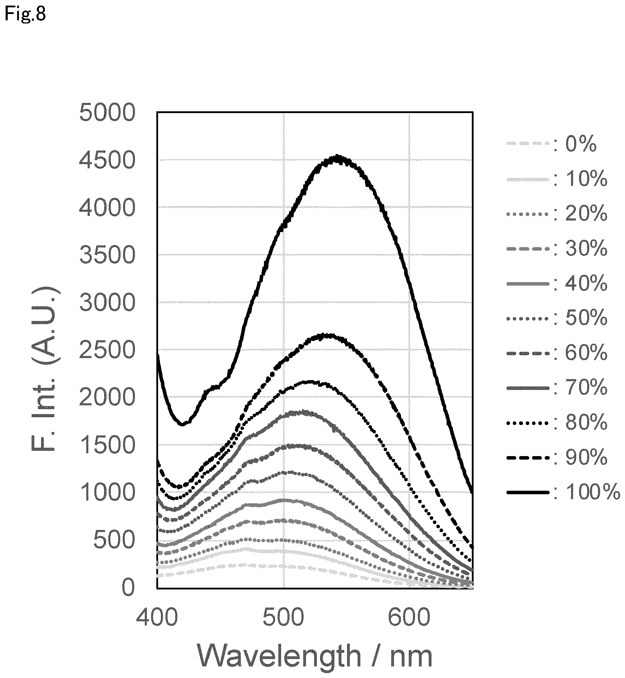

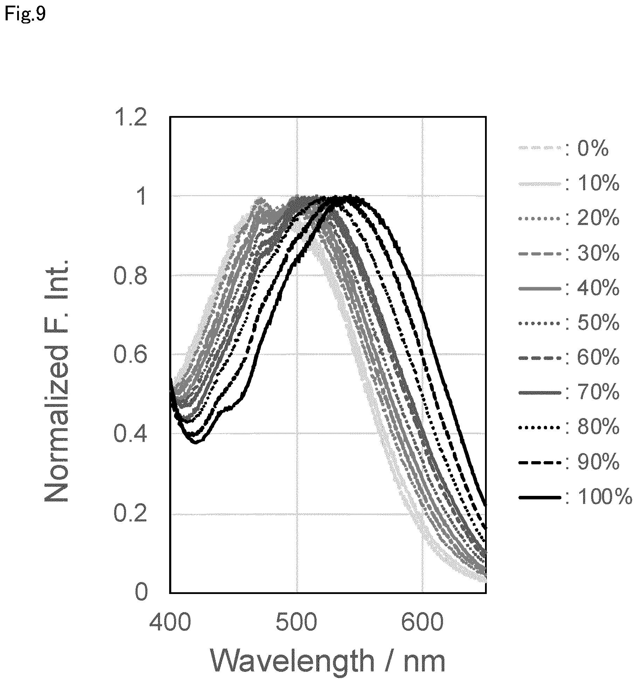

FIG. 8 and FIG. 9 each show a measurement result of excitation spectra (detection wavelength: 680 nm) in the first experiment. In FIG. 8, the vertical axis represents fluorescence intensity, and the horizontal axis represents wavelength. FIG. 9 is obtained by normalizing the fluorescence intensity such that the local maximum value of the fluorescence intensity for each viscosity shown in FIG. 8 is 1. As shown in FIG. 9, when the mixing ratio of glycerol is increased, that is, in accordance with increase in the solvent viscosity, the excitation local maximum wavelength is shifted to a long wavelength. For example, when the mixing ratio of glycerol is 0% (low viscosity), the excitation local maximum wavelength was 470 nm, and when the mixing ratio of glycerol was 100% (high viscosity), the excitation local maximum wavelength was 540 nm.

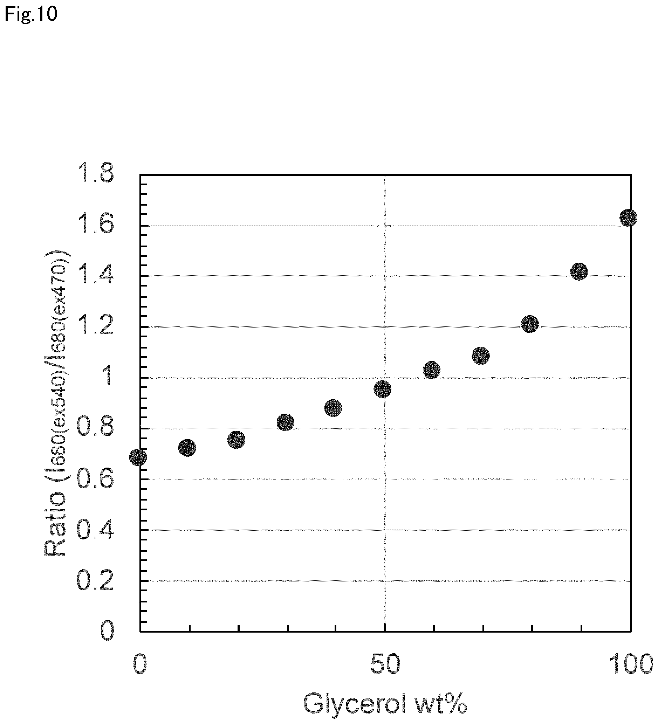

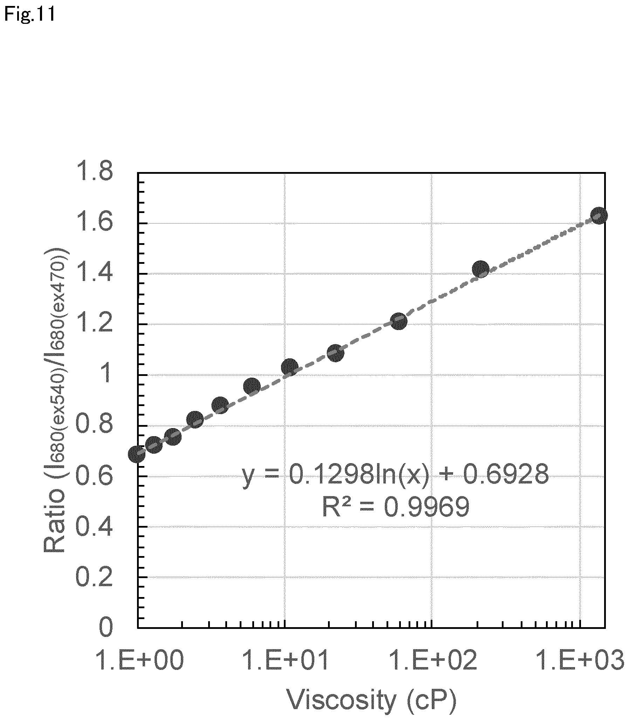

FIG. 10 and FIG. 11 each show correlation between a fluorescence intensity ratio and the solvent viscosity. In FIG. 10, the vertical axis represents fluorescence intensity ratio (I680 (ex540)/I680 (ex470)), and the horizontal axis represents mixing ratio of glycerol. FIG. 11 is obtained by representing the horizontal axis in FIG. 10 in terms of viscosity (logarithm) of the solvent. As shown in FIG. 11, the fluorescence intensity ratio linearly changes with respect to the logarithm of the solvent viscosity (cP). Therefore, when the compound 10 is used, the viscosity (solvent viscosity) of the liquid in which the compound 10 is present can be measured by using the fluorescence intensity ratio as an index.

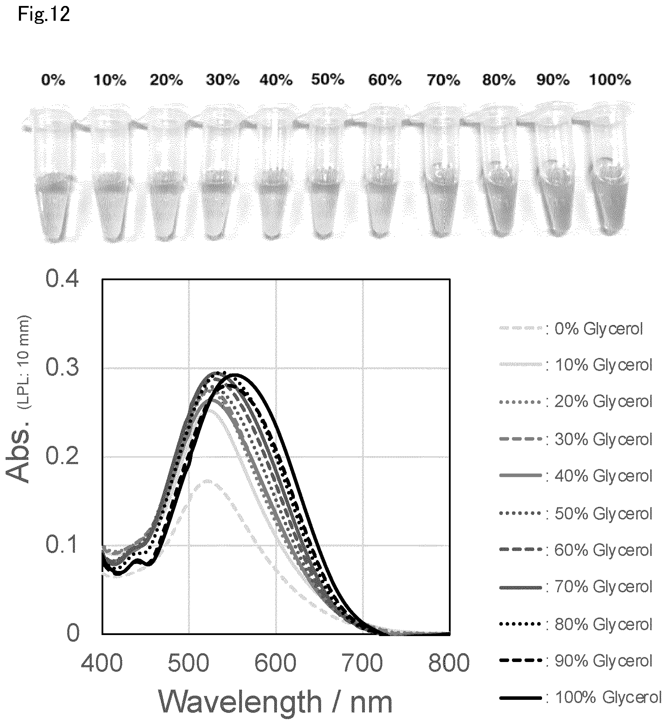

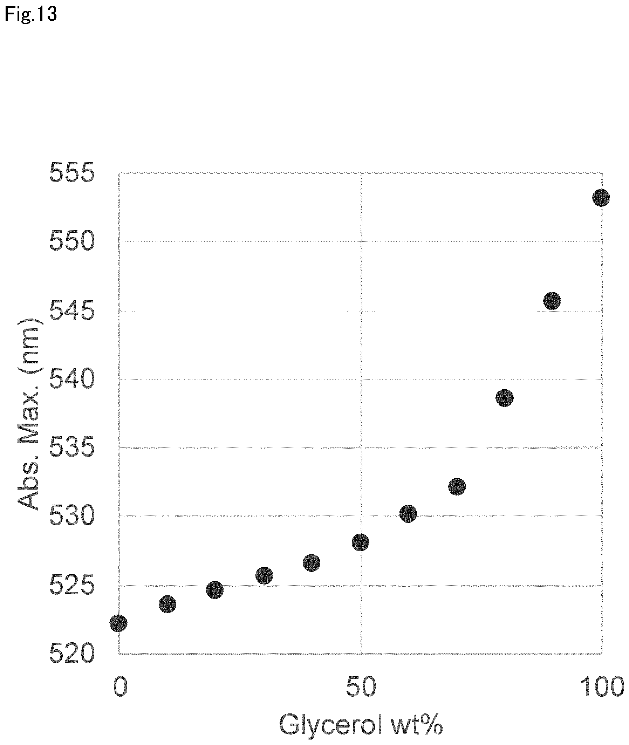

FIG. 12 shows the result of measurement of an absorption spectrum of each solvent containing the compound 10. In FIG. 12, the vertical axis represents absorbance and the horizontal axis represents wavelength. FIG. 13 shows the result of the measurement of the absorption spectrum, in terms of relationship between the absorption local maximum wavelength and the mixing ratio (viscosity) of glycerol. In FIG. 13, the vertical axis represents absorption local maximum wavelength, and the horizontal axis represents mixing ratio (viscosity) of glycerol. As shown in FIG. 13, in accordance with increase in the solvent viscosity, the absorption local maximum wavelength is shifted to a long wavelength. Therefore, when the compound 10 is used, the viscosity (solvent viscosity) of the liquid in which the compound 10 is present can be measured by using the absorption local maximum wavelength as an index.

As described above, with respect to the compound 10, fluorescence changes in accordance with the viscosity of the liquid in which the compound 10 is present. Therefore, when the compound 10 is used, the viscosity of the liquid in which the compound 10 is present can be measured. In the viscosity measurement using the compound 10, at least any one of the fluorescence intensity, the fluorescence wavelength, the fluorescence intensity ratio, and the absorbance which change in accordance with the viscosity is preferably measured as an index for obtaining the viscosity.

With respect to the compound 10, the fluorescence property such as the fluorescence intensity or the excitation wavelength changes in response to rotary motion of the three acceptor moieties. Therefore, when the three acceptor moieties can perform rotary motion, the viscosity measurement is possible. When the rotation speed is controlled according to the difference in the functional group introduced at R₁ or R₃, the range of viscosity change that can be measured with high accuracy can be controlled. For example, when -(CH₂CH₂0)n- is introduced as R₁ or R₃, the rotation speed can be controlled by changing the value of n.

### <3.2 Second experiment: change in fluorescence spectrum due to addition of DNA>

In the second experiment, a sample in which 6 µM of nucleic acid was added to 6 µM of the compound 10 synthesized as shown in "2.3 Production method of compound" and FIG. 4, was made. As the sample, a first sample, a second sample, and a third sample were made. The first sample was obtained by adding double-stranded DNA (ds(AAATTT)) containing an AAATTT sequence, to the compound 10. The second sample was obtained by adding double-stranded DNA (ds(AAGCTT)) not containing the AAATTT sequence, to the compound 10. The third sample was obtained by adding quadruplex DNA (Telo_G4) containing a telomere sequence. Further, the compound 10 to which no nucleic acid was added was used as a fourth sample. Each sample contained 50 mM of a tris-hydrochloric acid buffer (Tris-HCl) and 100 mM of potassium chloride (KCL).

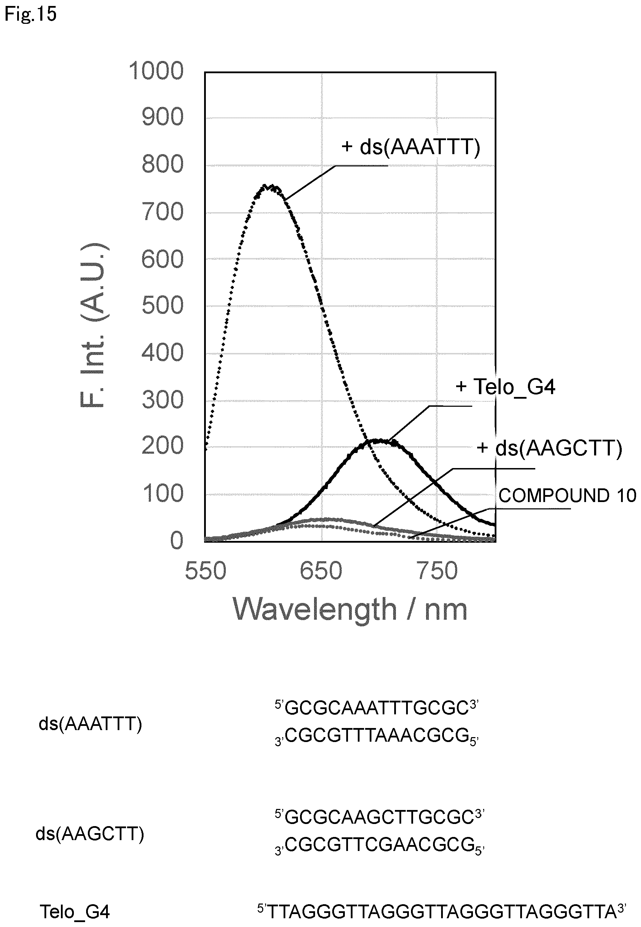

In the second experiment, a fluorescence spectrum (excitation wavelength: 470 nm) of each of the first sample, the second sample, the third sample, and the fourth sample was measured. FIG. 15 shows the measurement result. In FIG. 15, the vertical axis represents fluorescence intensity, and the horizontal axis represents wavelength.

As shown in FIG. 15, in the case of the first sample in which ds(AAATTT) was added, a fluorescence spectrum having a local maximum at 600 nm was observed, and the fluorescence intensity (600 nm) increased 38 times when compared with that of the fourth sample (the compound 10). Therefore, it is understood that the compound 10 binds to ds(AAATTT), whereby the fluorescence intensity increases. In the case of the second sample in which ds(AAGCTT) not containing the AAATTT sequence was added, the fluorescence intensity (600 nm) scarcely increased when compared with that of the fourth sample (the compound 10). Therefore, it is understood that, similar to QCy-DT, the compound 10 has sequence selectivity, and specifically binds to a double-stranded nucleic acid having an AAATTT sequence.

As shown in FIG. 15, in the case of the third sample in which Telo_G4 was added, a fluorescence spectrum having a local maximum at 670 nm was observed, and the fluorescence intensity (670 nm) increased 6 times when compared with that of the fourth sample (the compound 10). Therefore, it is understood that the compound 10 binds to Telo_G4, whereby the fluorescence intensity increases.

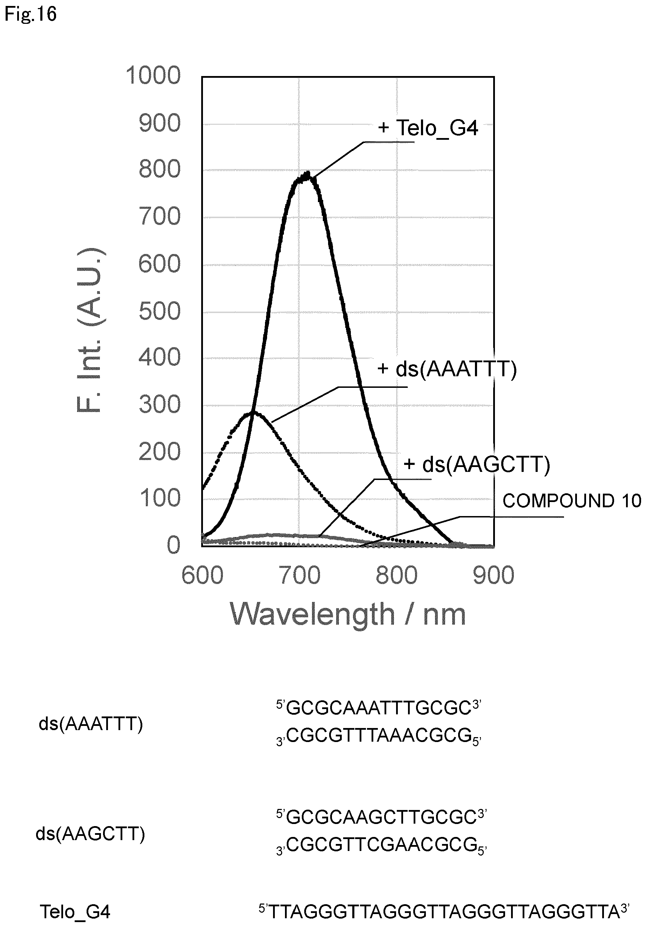

FIG. 16 shows the result of measurement of the fluorescence spectrum of each of the first sample, the second sample, the third sample, and the fourth sample, with the excitation wavelength changed to 570 nm.

As shown in FIG. 16, in the case of the first sample in which ds(AAATTT) was added, a fluorescence spectrum having a local maximum at 650 nm was observed, and the fluorescence intensity (650 nm) increased 27 times when compared with that of the fourth sample (the compound 10). In the case of the second sample in which ds(AAGCTT) not containing the AAATTT sequence was added, the fluorescence intensity (650 nm) scarcely increased when compared with that of the fourth sample (the compound 10).

As shown in FIG. 16, in the case of the third sample in which Telo_G4 was added, a fluorescence spectrum having a local maximum at 700 nm was observed, and the fluorescence intensity (700 nm) increased 150 times when compared with that of the fourth sample (the compound 10).

Thus, when the compound 10 is used as a fluorescent probe (fluorescent dye), a double-stranded nucleic acid can be detected, similar to QCy-DT. The compound 10 can specifically bind to a double-stranded nucleic acid having an AAATTT sequence, similar to QCy-DT. Further, when the compound 10 is used as a fluorescent probe (fluorescent dye), a quadruplex nucleic acid can be detected.

In addition, with respect to the compound 10, the wavelength at which the fluorescence intensity increases when the compound 10 binds to a double-stranded nucleic acid, and the wavelength at which the fluorescence intensity increases when the compound 10 binds to a quadruplex nucleic acid are different from each other. Therefore, based on the difference in the fluorescence wavelength, whether the DNA structure included in the sample is a double-stranded nucleic acid or a quadruplex nucleic acid can be determined.

The compound 10 can also be used in viscosity measurement as described above. Therefore, detection of a nucleic acid and measurement of the viscosity of the environment in which the nucleic acid is present can be simultaneously performed. For example, when the compound 10 has bound to a quadruplex nucleic acid, at least any one of the fluorescence intensity, the fluorescence wavelength, the fluorescence intensity ratio, and the absorbance changes in accordance with the magnitude of the viscosity in the environment in which the quadruplex nucleic acid is present.

Therefore, for example, from the fluorescence spectrum or the like obtained by measurement of a sample containing the compound 10, whether the DNA structure contained in the sample is a double-stranded nucleic acid or a quadruplex nucleic acid can be determined, and the viscosity of the environment in which the nucleic acid is present can be obtained.

### <3.3 Third experiment: affinity of interaction of compound 10 with quadruplex nucleic acid and double-stranded nucleic acid>

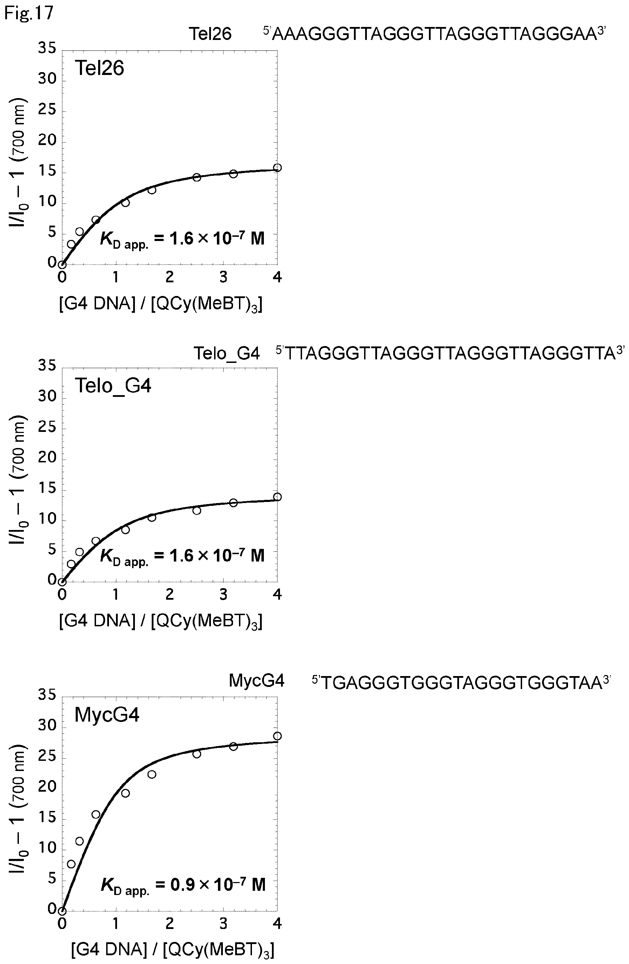

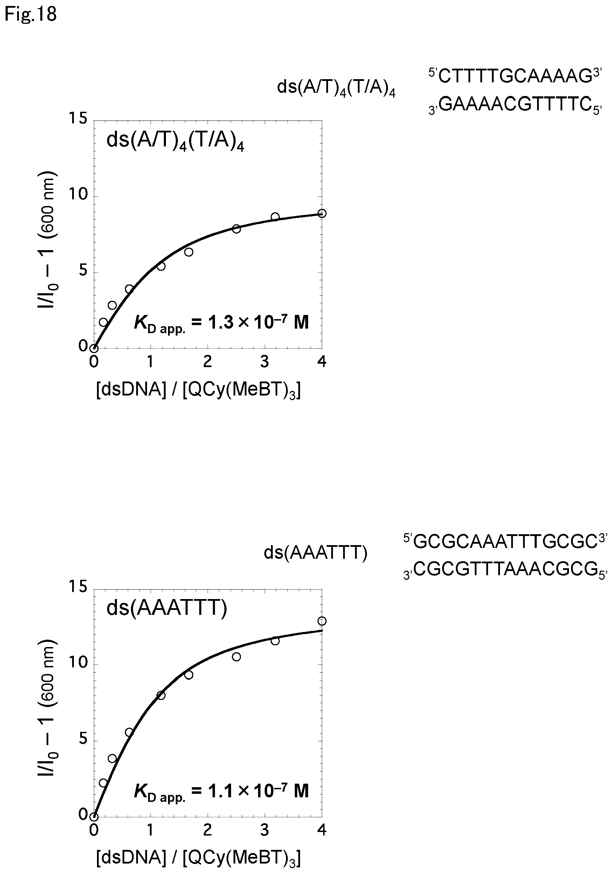

In the third experiment, in order to evaluate the binding affinity between the compound 10 synthesized as shown in "2.3 Production method of compound" and FIG. 4, and a quadruplex nucleic acid or a double-stranded nucleic acid, a fluorometric titration experiment was performed, and a dissociation constant K_{D} was calculated. The dissociation constant K_{D} was calculated according to nonlinear least squares fitting, based on a titration curve obtained through the fluorometric titration experiment. In the third experiment, the dissociation constant between the compound 10 and Tel26 being a G4 DNA, the dissociation constant between the compound 10 and Telo_G4 being a G4 DNA, the dissociation constant between the compound 10 and MycG4 being a G4 DNA, the dissociation constant between the compound 10 and ds(A/T)₄(T/A)₄ being a dsDNA, and the dissociation constant between the compound 10 and ds(AAATTT) being a dsDNA were obtained. FIG. 17 and FIG. 18 show the result. FIG. 17 and FIG. 18 show fluorescence intensity change when a quadruplex nucleic acid or a double-stranded nucleic acid was added to the compound 10. In FIG. 17 and FIG. 18, I included in the vertical axis is fluorescence intensity, and I₀ also included in the vertical axis is fluorescence intensity when no quadruplex nucleic acid or no double-stranded nucleic acid was added. In FIG. 17 and FIG. 18, the horizontal axis represents concentration ratio between the compound 10 and a double-stranded nucleic acid or a quadruplex nucleic acid. In FIG. 17 and FIG. 18, the compound 10 is indicated as QCy(MeBT)₃.

With respect to the binding affinity between the compound 10 and the quadruplex nucleic acid, as shown in FIG. 17, the dissociation constant between the compound 10 and Tel26 was 1.6×10⁻⁷ M, the dissociation constant between the compound 10 and Telo_G4 was 1.6×10⁻⁷ M, and the dissociation constant between the compound 10 and MycG4 was 0.9×10⁻⁷ M. The smaller the dissociation constant is, the larger the binding strength is.

According to the result shown in FIG. 17, the compound 10 has a binding affinity equivalent to or larger than that of a conventional quadruplex nucleic acid ligand. That is, the dissociation constant between the conventional quadruplex nucleic acid ligand and a quadruplex nucleic acid is about 1.0 to 10×10⁻⁶. The dissociation constant between the compound 10 and a quadruplex nucleic acid is about 0.9 to 1.6×10⁻⁷, and thus, the compound 10 has a binding affinity equivalent to or larger than that of the conventional quadruplex nucleic acid ligand.

With respect to the binding affinity between the compound 10 and a double-stranded nucleic acid, as shown in FIG. 18, the dissociation constant between the compound 10 and ds(A/T)₄(T/A)₄ was 1.3×10⁻⁷ and the dissociation constant between the compound 10 and ds(AAATTT) was 1.1×10⁻⁷.

According to the result shown in FIG. 18, the compound 10 has a larger binding strength to a double-stranded nucleic acid than that of QCy-DT being a conventional fluorescent ds probe. That is, the dissociation constant between QCy-DT and a double-stranded nucleic acid (ds(AAATTT)) is about 6.7×10⁻⁷, which is relatively large, and thus, the binding affinity of QCy-DT is relatively small. However, the dissociation constant between the compound 10 and a double-stranded nucleic acid is about 1.1 to 1.3×10⁻⁷, which is relatively small, and thus, the binding affinity of the compound 10 is relatively large.

Since the compound 10 has a relatively large binding affinity to a nucleic acid, the compound 10 is advantageous as a fluorescent probe for the nucleic acid. It is considered that since the compound 10 has three legs positively charged, the binding force to a nucleic acid negatively charged is large.

### <3.4 Fourth experiment: two-color fluorescence imaging of quadruplex nucleic acid and double-stranded nucleic acid in fixed cell using compound 10>

In a fourth experiment, quadruplex nucleic acids and double-stranded nucleic acids in fixed cells were stained, using the compound 10 synthesized as shown in "2.3 Production method of compound" and FIG. 4. In the fourth experiment, HeLa cells having been monolayer-cultured were subjected to a fixation process, and washed with phosphate buffered saline (PBS). The fixation process was performed by immersing the HeLa cells in a 25% (V/V) acetic acid/methanol solution for 10 minutes at room temperature. Then, a PBS solution (6 µM) of the compound 10 was added to the HeLa cells, and the resultant matter was left for 10 minutes, and then was subjected to, as they were without being washed, fluorescence microscopy (Ex 470/40, DM 495, Em 605/70; Ex 560/40, DM 585, Em 700/75).

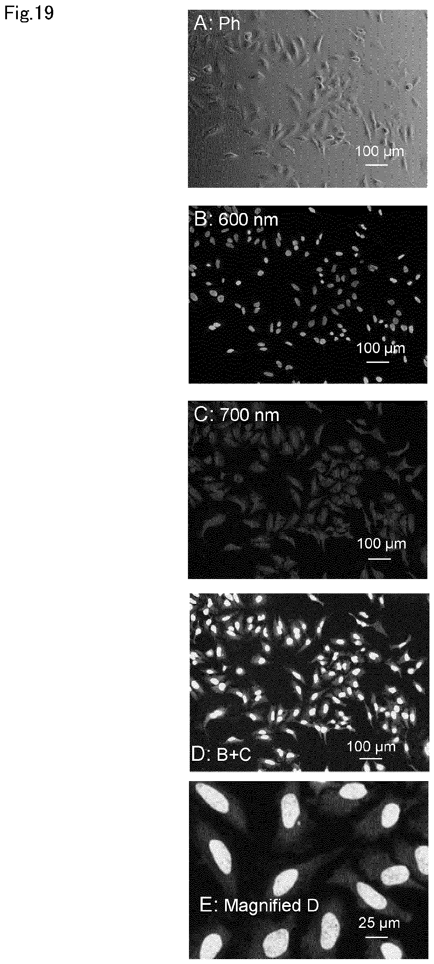

FIG. 19 shows the result of the fourth experiment. In FIG. 19, "A" is a phase contrast observation image (Ph) of cells. The fluorescence image of "B" shows that double-stranded DNAs present in the cell nuclei of the cells shown in "A" are stained by 600 nm fluorescence. The fluorescence image of "C" shows that quadruplex DNAs and quadruplex RNAs present in the cell nuclei of the cells shown in "A" are stained by 700 nm fluorescence. Thus, when the compound 10 is used, both of quadruplex nucleic acids and double-stranded nucleic acids in fixed cells can be stained simultaneously and by fluorescences of different colors. "D" in FIG. 19 is obtained by superposing "B" and "C", and "E" is a magnified image of "D".

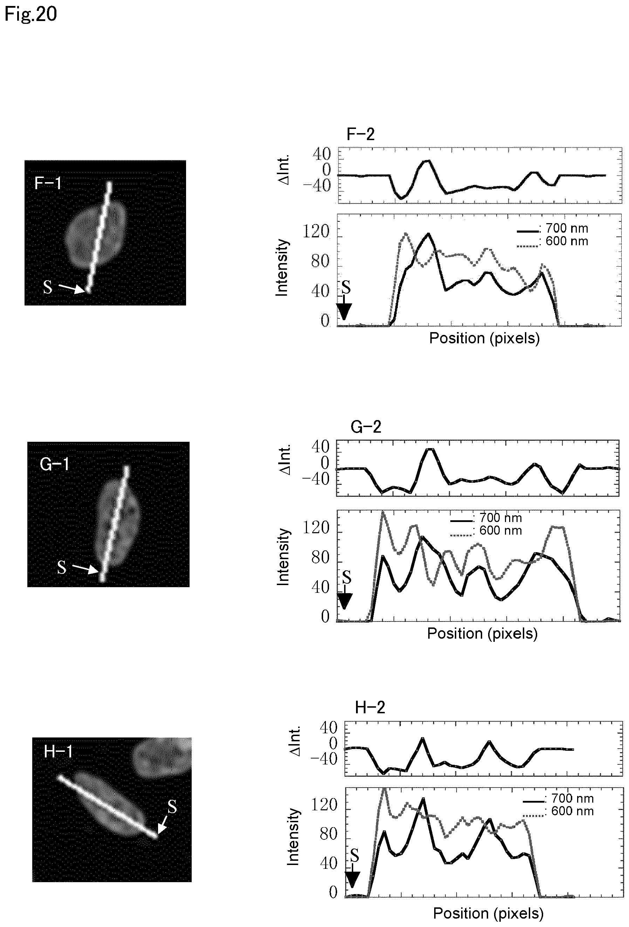

FIG. 20 shows intensity (Intensity) of each of 700 nm fluorescence and 600 nm fluorescence and intensity difference (Δint.) in three cells (F-1, G-1, and H-1) arbitrarily picked from "E" in FIG. 19. In F-2, G-2, and H-2 in FIG. 20, "Position" in the horizontal axis indicates the position along a white line, with reference to position S in F-1, G-1, and H-1.

As shown in FIG. 20, portions where the 700 nm fluorescence was strong were observed in the cell nucleus. Therefore, it was clarified that the quadruplex DNA in the nucleus could be detected. In FIG. 20, the difference in the intensity (Intensity) of each of the 700 nm fluorescence and the 600 nm fluorescence, and the intensity difference (Δint.) correspond to the amounts of the quadruplex nucleic acid and the double-stranded nucleic acid in the cell. Therefore, the place where the quadruplex nucleic acid is present in abundance can be specified based on the intensity (Intensity) of each of the 700 nm fluorescence and the 600 nm fluorescence.

### <3.5 Fifth experiment: evaluation of cytotoxicity of compound 10>

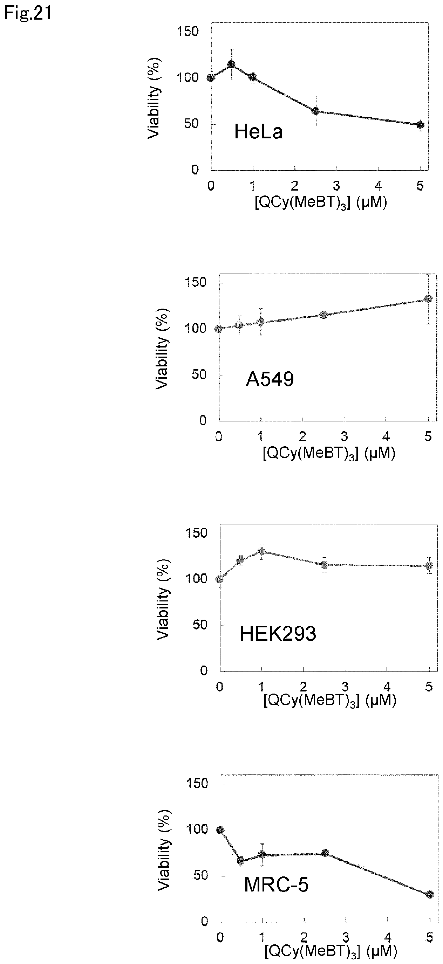

In a fifth experiment, cytotoxicity of the compound 10 synthesized as shown in "2.3 Production method of compound" and FIG. 4 was evaluated. In the fifth experiment, a 5% glucose aqueous solution (0 µM, 0.5 µM, 1 µM, 2.5 µM, 5 µM) of the compound 10 was added to each of HeLa cells, A549 cells, HEK293 cells, and MRC-5 cells, which were monolayer-cultured, and the resultant matter was incubated for 10 minutes. After washing, the viable cell percentage was measured using a commercially-available cell count colorimetry kit. FIG. 21 shows the result. In FIG. 21, the horizontal axis represents concentration (µM) of the compound 10, and the vertical axis represents viability of cells. In FIG. 21, the compound 10 is indicated as QCy(MeBT)₃.

As shown in FIG. 21, it was found that, when the concentration of the compound 10 was not higher than 1 µM, a viable cell percentage of not lower than 70% was maintained in all cell strains. Therefore, for viable cells, the compound 10 is preferably used in a concentration of not higher than 1 µM.

### <3.6 Sixth experiment: two-color fluorescence imaging of quadruplex nucleic acid and double-stranded nucleic acid in viable cells using compound 10>

In a sixth experiment, quadruplex nucleic acids and double-stranded nucleic acids in viable cells were stained, using the compound 10 synthesized as shown in "2.3 Production method of compound" and FIG. 4. In the fourth experiment, the compound 10 was dissolved in a 5% glucose aqueous solution, and the resultant matter was used as a staining liquid. The concentration of the compound 10 in the staining liquid was set to be 1 µM. The culture medium was removed from the cultured cells (HeLa cells), and the resultant matter was washed with a 5% glucose aqueous solution. Then, the staining liquid containing the compound 10 was added thereto, and the resultant matter was incubated at 37°C for 10 minutes. The resultant matter was washed with a 5% glucose aqueous solution, and then subjected to fluorescence microscopy (Ex 470/40, DM 495, Em 605/70; Ex 560/40, DM 585, Em 700/75).

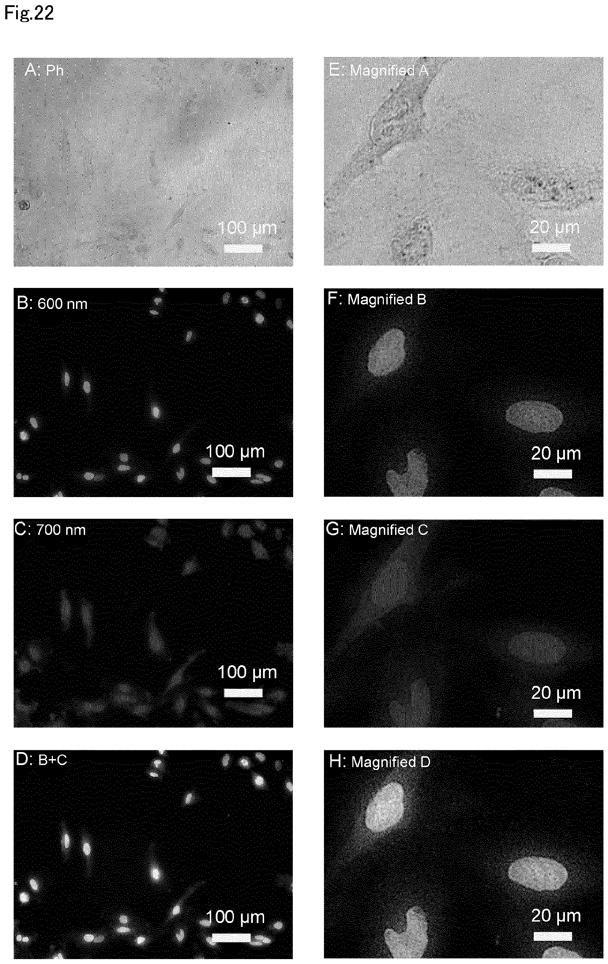

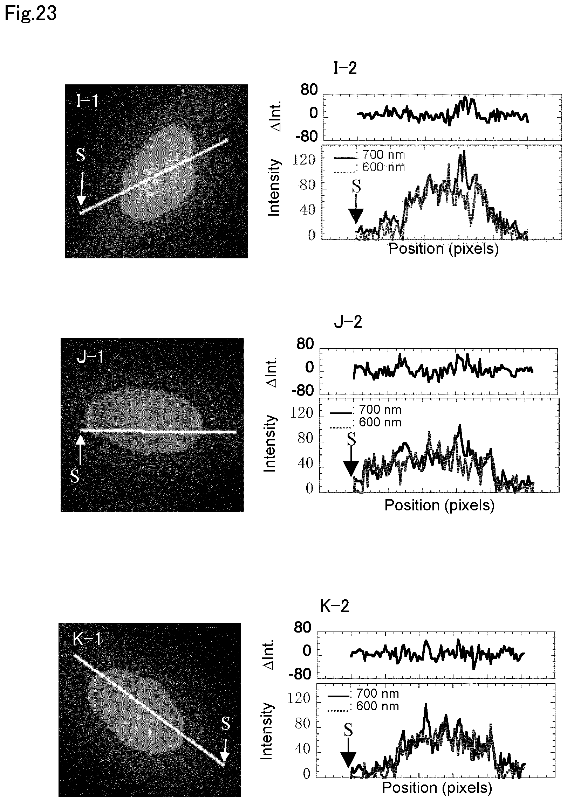

FIG. 22 and FIG. 23 show the result of the sixth experiment. In FIG. 22, "A" is a phase contrast observation image (Ph) of cells. The fluorescence image of "B" shows that double-stranded DNAs present in the cell nuclei of the cells shown in "A" are stained by 600 nm fluorescence. The fluorescence image of "C" shows that quadruplex DNAs and quadruplex RNAs present in the cell nuclei of the cells shown in "A" are stained by 700 nm fluorescence. In FIG. 22, "D" is obtained by superposing "B" and "C", "E" is a magnified image of "A", "F" is a magnified image of "B", "G" is a magnified image of "C", and "H" is a magnified image of "D". As shown in FIG. 22, when the compound 10 is used, also with respect to viable cells, similar to fixed cells, both of quadruplex nucleic acids and double-stranded nucleic acids in viable cells can be stained simultaneously and by fluorescences of different colors.

FIG. 23 shows intensity (Intensity) of each of 700 nm fluorescence and 600 nm fluorescence and intensity difference (Δint.) in three cells (I-1, J-1, and K-1) arbitrarily picked from "D" in FIG. 22. In I-2, J-2, and K-2 in FIG. 23, "Position" in the horizontal axis indicates the position along a white line, with reference to position S in I-1, J-1, and K-1.

As shown in FIG. 23, portions where the 700 nm fluorescence was strong were observed in the cell nucleus. Therefore, it was clarified that the quadruplex DNA in the nucleus could be detected.

### <3.7 Seventh experiment: tracking of kinetics of quadruplex nucleic acid in viable cell using compound 10>

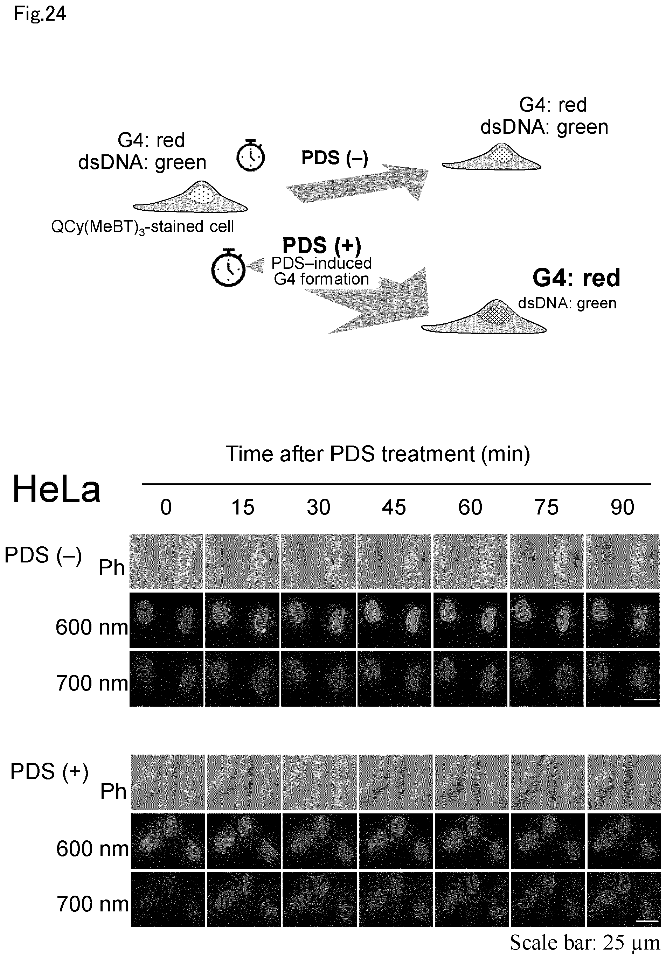

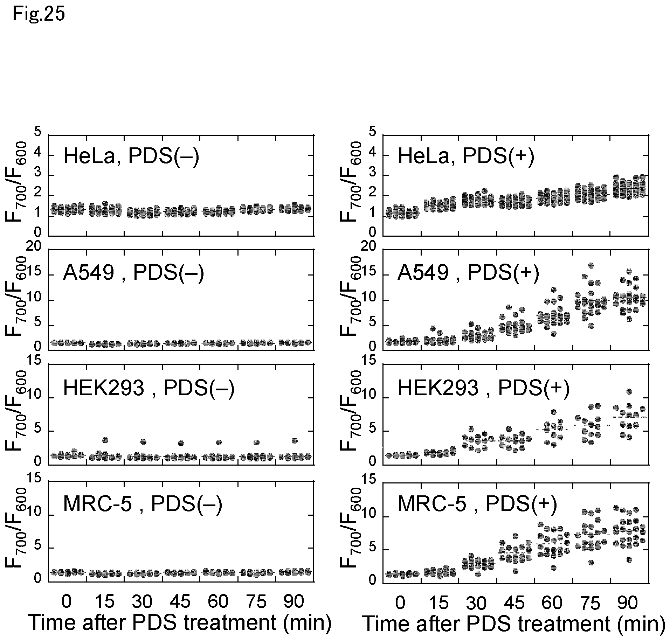

In the seventh experiment, it was demonstrated that imaging of the formation process of a quadruplex nucleic acid in a viable cell is possible. In the seventh experiment, pyridostatin (PDS; 20 µM) was added to viable cells stained by the same method as that in the sixth experiment and temporal change of a fluorescence image thereafter was observed. Pyridostatin (PDS) is a reagent for stabilizing quadruplex nucleic acids. FIG. 24 and FIG. 25 show the result of the seventh experiment.

FIG. 24 shows temporal change in phase contrast observation images (Ph) and fluorescence images (600 nm fluorescence image, 700 nm fluorescence image), in a case (PDS (+)) where PDS was added to HeLa cells (viable cells) stained by the compound 10, and a case (PDS (-)) where PDS was not added.

FIG. 25 shows the response (fluorescence intensity change) to PDS of each of four types of viable cells (HeLA, A549, HEK293, MRC-5) stained by the compound 10. In FIG. 25 as well, a case where PDS was added to the viable cells stained by the compound 10 was indicated with PDS (+), and a case where PDS was not added was indicated with PDS (-). In FIG. 25, the vertical axis represents intensity ratio between 600 nm fluorescence and 700 nm fluorescence, and the horizontal axis represents time after addition of PDS.

As shown in FIG. 25, it is seen that, in the case (PDS (+)) where PDS was added, the intensity ratio of 700 nm fluorescence to 600 nm fluorescence increased. Meanwhile, when PDS was not added, the fluorescence intensity ratio scarcely changed. Therefore, when viable cells are stained by the compound 10, the process of formation of quadruplex nucleic acids in the viable cells can be imaged or visualized.

### <3.8 Eighth experiment: examination of solvent for staining liquid (probe solution)>

As a solvent for the staining liquid (probe solution) having the compound 10, a liquid, such as H₂0 or 5% glucose, that does not substantially have ions that bind to the compound 10 is preferable. In a case of a solvent (e.g., PBS) having ions that bind to the compound 10, even if the compound 10 is dissolved in the solvent, precipitation occurs with lapse of time. However, in a case of a solvent that does not substantially have ions that bind to the compound 10, precipitation can be prevented. Further, as the solvent, one, such as 5% glucose, whose osmotic pressure is substantially equal to that of a cell is preferable. When the osmotic pressure is substantially equal to that of a cell, the cell can be protected.

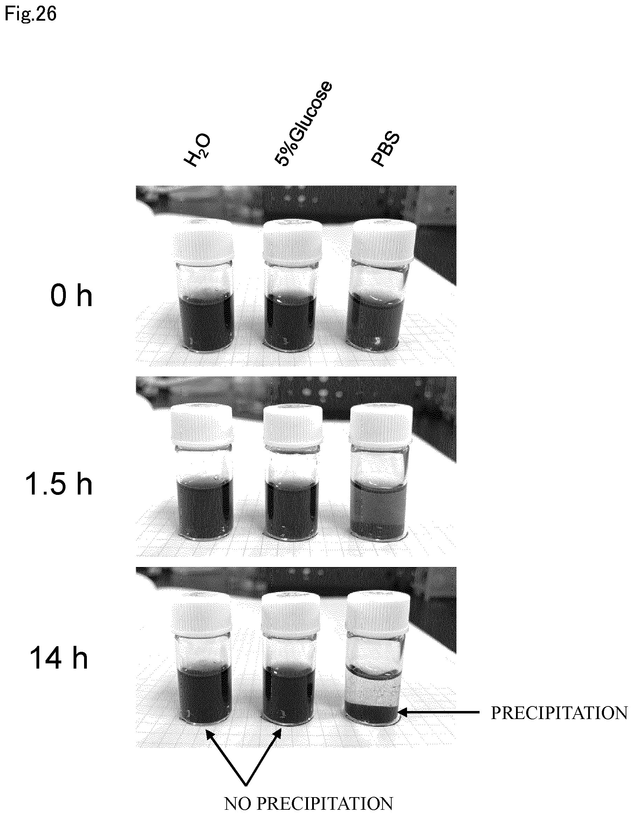

In the eighth experiment, the compound 10 (5 µM) was dissolved into each of solvents of H₂0, 5% glucose, and PBS, and the resultant matter was left to stand at room temperature. FIG. 26 shows the scene. When the compound 10 was dissolved in PBS, precipitation occurred with lapse of time, and substantially all of the precipitate sank after 14 hours. Meanwhile, in the case of H₂0 or 5% glucose, precipitation did not occur even after 14 hours. As shown in the eighth experiment, when a solvent that does not substantially have ions that bind to the compound 10 is adopted, precipitation can be prevented.

### <3.9 Ninth experiment: competition experiment with Hoechist 33258 (using fixed HeLa cells)>

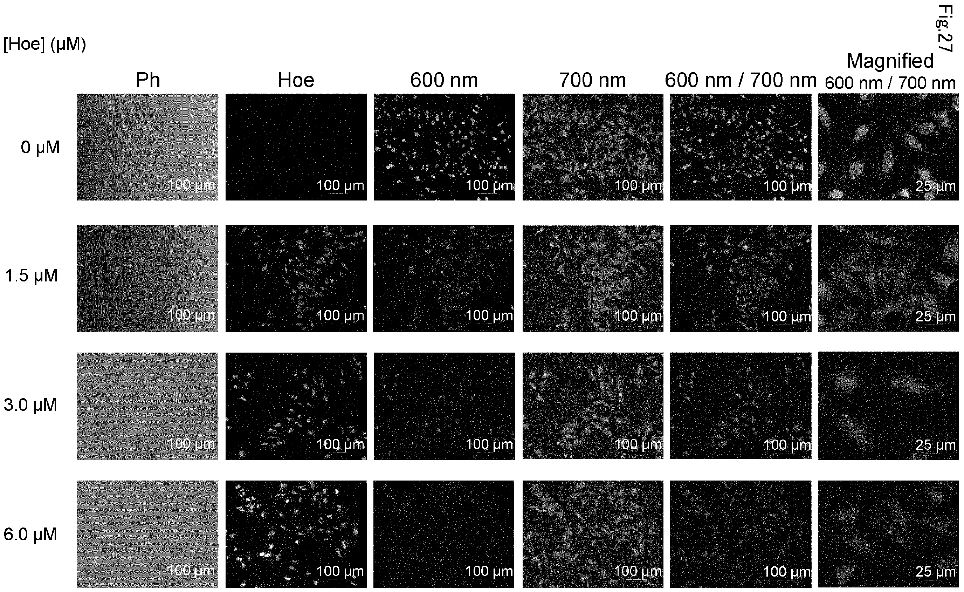

In a ninth experiment, a competition experiment with Hoechist 33258 was performed using fixed HeLa cells. As the staining liquid, one obtained by causing Hoechist 33258 at an arbitrary concentration (0 µM, 1.5 µM, 3.0 µM, 6.0 µM) to coexist in a PBS solution of the compound 10 (6 µM) was used, and the experiment was performed according to an operation similar to that in the fourth experiment. FIG. 27 shows the result. In FIG. 27, "Ph" is a phase contrast observation image of cells at each concentration of Hoechist 33258. "Hoe" is a 460 nm fluorescence image at each concentration of Hoechist 33258. The fluorescence wavelength of Hoechist 33258 is 460 nm. "600 nm" is a 600 nm fluorescence image. "700 nm" is a 700 nm fluorescence image. "600 nm/700 nm" is a synthesized image of "600 nm" and "700 nm". "Magnified 600 nm/700 nm" is a magnified image of "600 nm/700 nm".

As shown in FIG. 27, in association with increase in the addition concentration of Hoechist 33258 selectively binding to a double-stranded DNA, 600 nm fluorescence intensity in the fixed cells significantly decreased. This indicates that the binding sites of the compound 10 and Hoechist 33258 compete with each other. That is, it was found that the compound 10 binds to a double-stranded DNA similar to Hoechist 33258, and emits 600 nm fluorescence. Therefore, it was clarified that, when 600 nm fluorescence derived from the compound 10 was observed, double-stranded DNAs in the fixed cells were able to be visualized.

### <3.10 Tenth experiment: competition experiment with Hoechist 33258 (using living HeLa cells)>

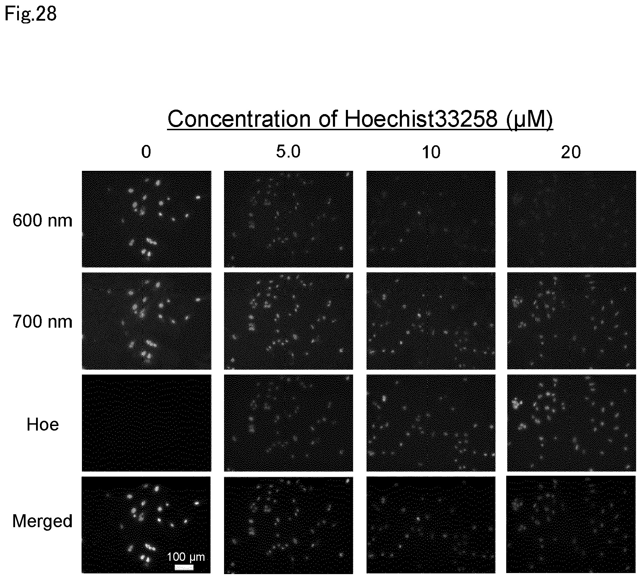

In a tenth experiment, a competition experiment with Hoechist 33258 was performed using living HeLa cells. As the staining liquid, one obtained by causing Hoechist 33258 at an arbitrary concentration (0 µM, 5.0 µM, 10 µM, 20 µM) to coexist in a 5% glucose solution of the compound 10 (1 µM) was used, and the experiment was performed according to an operation similar to that in the sixth experiment. FIG. 28 shows the result. In FIG. 28, "600 nm" is a 600 nm fluorescence image. "700 nm" is a 700 nm fluorescence image. "Hoe" is a 460 nm fluorescence image at each concentration of Hoechist 33258. "Merged" is a synthesized image of "600 nm", "700 nm", and "Hoe".

As shown in FIG. 28, in association with increase in the addition concentration of Hoechist 33258 selectively binding to a double-stranded DNA, 600 nm fluorescence intensity in the viable cells significantly decreased. This indicates that the binding site of the compound 10 and Hoechist 33258 compete with each other. That is, it was found that the compound 10 binds to a double-stranded DNA similar to Hoechist 33258, and emits 600 nm fluorescence. Therefore, it was clarified that, when 600 nm fluorescence derived from the compound 10 was observed, double-stranded DNAs in the viable cells were able to be visualized.

### <4. Additional note>

The present invention is not limited to the above embodiment and examples, and various modifications can be made.

### REFERENCE SIGNS LIST

10 compound (QCy(BT)₃)
11 donor
12A first acceptor
12B second acceptor
13C third acceptor
100 compound (QCy-DT)
111 donor
112A acceptor
112B acceptor

## Claims

1. A method comprising:
using a compound having a structure represented by general formula (1A) or general formula (1B), for nucleic acid detection, 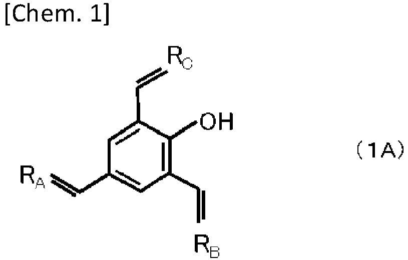 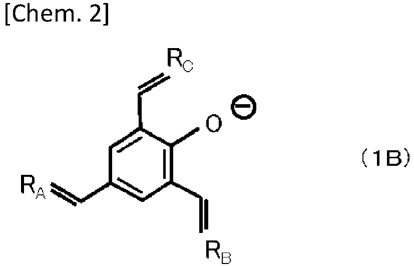 where
R_{A}, R_{B}, and R_{C} each independently have a structure selected from the group consisting of formula (1C), formula (1D), formula (1E), and formula (1F) below (* representing a binding site), 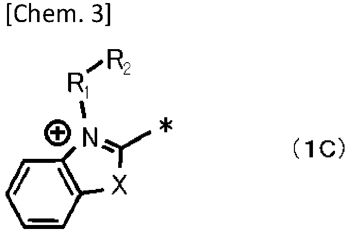 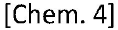 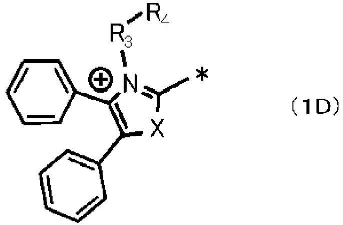 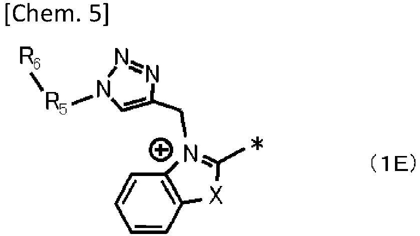 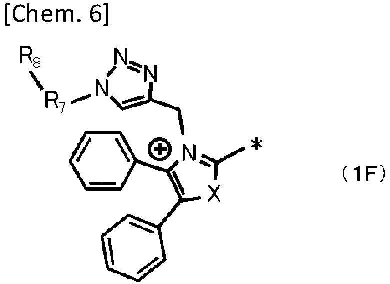
X is selected from the group consisting of O, S, and Se,
R₁, R₃, R₅, and R₇ are each independently selected from the group consisting of H, -(CH₂)n-, and -(CH₂CH₂O)n-, n being a natural number of 1 to 100, and
R₂, R₄, R₆, and R₈ are each independently selected from the group consisting of H, -OH, methyl, an amine, a terminal alkyne, an alkene, an alkyl acid, an amine acid, sulfonate (SO₃⁻), an aryl group or a derivative thereof, and a heterocyclic compound or a derivative thereof.

2. The method according to claim 1, comprising
using the compound for detection of at least either one of a double-stranded nucleic acid and a quadruplex nucleic acid.

3. The method according to claim 1, comprising
using the compound for nucleic acid detection in which a double-stranded nucleic acid and a quadruplex nucleic acid are distinguished from each other.

4. The method according to any one of claims 1 to 3, comprising
using the compound for detection of a viscosity of a sample containing a nucleic acid.

5. The method according to any one of claims 1 to 4, wherein
X is S.

6. A compound having a structure represented by general formula (1A) or general formula (1B), 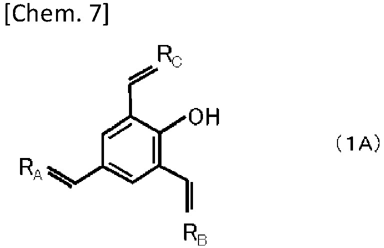 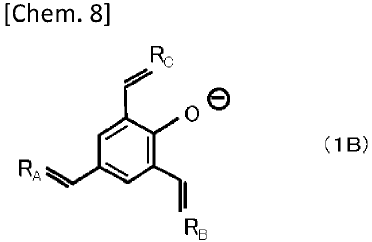 where
R_{A}, R_{B}, and R_{C} each independently have a structure selected from the group consisting of formula (1C), formula (1D), formula (1E), and formula (1F) below (* representing a binding site), 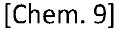 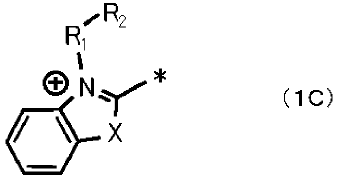 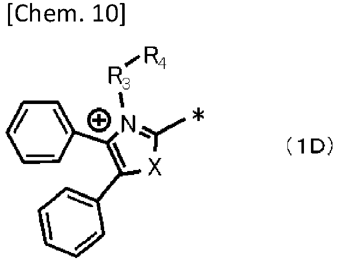 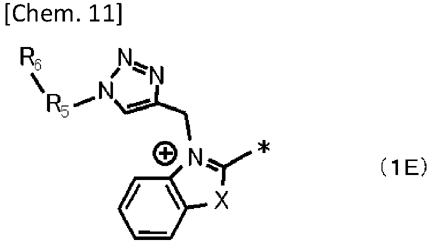 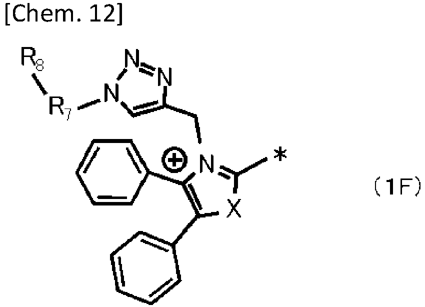
X is selected from the group consisting of O, S, and Se,
R₁, R₃, R₅, and R₇ are each independently selected from the group consisting of H, -(CH₂)n-, and -(CH₂CH₂O)n-, n being a natural number of 1 to 100, and
R₂, R₄, R₆, and R₈ are each independently selected from the group consisting of H, -OH, methyl, an amine, a terminal alkyne, an alkene, an alkyl acid, an amine acid, sulfonate (SO₄⁻), an aryl group or a derivative thereof, and a heterocyclic compound or a derivative thereof.

7. A fluorescent probe comprising
the compound according to claim 6.

8. A method comprising
using the compound according to claim 6 for measurement of a viscosity of a liquid.

9. A compound comprising:
a donor; and
three acceptors bound to the donor so as to perform rotary motion with respect to the donor, wherein
a fluorescence property of the compound changes in accordance with change in the rotary motion of each acceptor.

10. The compound according to claim 9, capable of binding to at least either one of a double-stranded nucleic acid and a quadruplex nucleic acid.

11. The compound according to claim 9, capable of binding to both of a double-stranded nucleic acid and a quadruplex nucleic acid.

12. The compound according to claim 11, wherein
when any two of the three acceptors have bound to a minor groove of the double-stranded nucleic acid, a fluorescence intensity having a first wavelength becomes higher than that when the two acceptors are not bound to the double-stranded nucleic acid, and
when the three acceptors have bound to the quadruplex nucleic acid, a fluorescence intensity having a second wavelength different from the first wavelength becomes higher than that when the three acceptors are not bound to the quadruplex nucleic acid.

13. A method comprising
using the compound according to any one of claims 9 to 12, for nucleic acid detection.

14. A method comprising
using the compound according to any one of claims 9 to 12, for nucleic acid detection in which a double-stranded nucleic acid and a quadruplex nucleic acid are distinguished from each other.

15. A method comprising
using the compound according to any one of claims 9 to 12, for measurement of a viscosity of a liquid.
